(19) Europäisches Patentamt
European Patent Office
Office européen des brevets

(11) EP 2 029 504 B1

(12) # EUROPÄISCHE PATENTSCHRIFT

(45) Veröffentlichungstag und Bekanntmachung des Hinweises auf die Patenterteilung:
**10.08.2016 Patentblatt 2016/32**

(21) Anmeldenummer: **07729926.1**

(22) Anmeldetag: **06.06.2007**

(51) Int Cl.:
***C07C 2/12*** *(2006.01)*

(86) Internationale Anmeldenummer:
**PCT/EP2007/055551**

(87) Internationale Veröffentlichungsnummer:
**WO 2007/141288 (13.12.2007 Gazette 2007/50)**

(54) **VERFAHREN ZUR CO-DIMERISIERUNG VON OLEFINEN**

PROCESS FOR CODIMERIZING OLEFINS

PROCEDE DE CO-DIMERISATION D'OLEFINES

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **07.06.2006 EP 06115091**

(43) Veröffentlichungstag der Anmeldung:
**04.03.2009 Patentblatt 2009/10**

(73) Patentinhaber: **BASF SE**
**67056 Ludwigshafen am Rhein (DE)**

(72) Erfinder:
- **SIGL, Marcus**
  **68167 Mannheim (DE)**
- **TRILLER, Michael**
  **68549 Ilvesheim (DE)**
- **HEIDEMANN, Thomas**
  **68519 Viernheim (DE)**

(56) Entgegenhaltungen:
**WO-A-00/56683**
**WO-A-01/36356**
**WO-A-01/83407**
**WO-A-03/082780**
**WO-A-2004/080935**
**WO-A-2007/040812**
**US-A- 5 118 901**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 029 504 B1

Printed by Jouve, 75001 PARIS (FR)

## Beschreibung

Die vorliegende Erfindung betrifft ein Verfahren zur Herstellung von Olefin-Co-Dimeren mit 6 bis 18 Kohlenstoffatomen, die nach diesem Verfahren erhältlichen Olefin-Co-Dimere, ein Verfahren zur Herstellung von Alkoholen, bei dem man solche Olefin-Co-Dimere einer Hydroformylierung und anschließenden Hydrierung unterzieht, die so erhältlichen Alkoholgemische und deren Verwendung.

Kohlenwasserstoffgemische, die kurzkettige Olefine, z. B. mit 2 bis 6 Kohlenstoffatomen enthalten, sind im großtechnischen Maßstab erhältlich. So fällt z. B. bei der Aufarbeitung von Erdöl durch Steamcracken oder Fluidized Catalyst Cracking (FCC) ein als $C_4$-Schnitt bezeichnetes Kohlenwasserstoffgemisch mit einem hohen Gesamtolefingehalt an, wobei es sich im Wesentlichen um Olefine mit 4 Kohlenstoffatomen handelt. Solche $C_4$-Schnitte, d. h. Gemische aus isomeren Butenen und Butanen, eignen sich, gegebenenfalls nach einer vorherigen Abtrennung des Isobutens und Hydrierung des enthaltenen Butadiens, sehr gut zur Herstellung von Oligomeren, insbesondere von Octenen und Dodecenen.

Eine große Bedeutung haben die aus Olefingemischen mit überwiegend linearen Ausgangsolefinen erhältlichen im Wesentlichen linearen Oligomerengemische erlangt. Sie eignen sich z. B. als Dieselkraftstoffkomponente sowie als Zwischenprodukte zur Herstellung funktionalisierter, überwiegend linearer Kohlenwasserstoffe. So erhält man durch Hydroformylierung und anschließende Hydrierung der Olefinoligomere die entsprechenden Alkohole, die unter anderem als Ausgangsstoffe für Detergenzien und als Weichmacher verwendet werden.

Es ist bekannt, Fettalkohole mit etwa 8 bis 20 Kohlenstoffatomen zur Herstellung von nichtionischen und anionischen Tensiden einzusetzen. Dazu werden die Alkohole einer entsprechenden Funktionalisierung, z. B. durch Alkoxylierung oder Glycosidierung, unterworfen. Die erhaltenen Alkoxylate können entweder direkt als nichtionische oberflächenaktive Substanzen eingesetzt oder durch eine weitere Funktionalisierung, z. B. durch Sulfatierung oder Phosphatierung, in anionische oberflächenaktive Substanzen überführt werden. Die anwendungstechnischen Eigenschaften dieser Tenside, z. B. deren Netzvermögen, Schaumbildung, Fettlösevermögen, biologische Abbaubarkeit usw., werden im Wesentlichen durch die Kettenlänge und den Verzweigungsgrad des hydrophoben Kohlenwasserstoffrestes des eingesetzten Alkohols bestimmt. Alkohole, die sich gut zur Weiterverarbeitung zu wirksamen Tensiden eignen, werden auch als Tensidalkohole bezeichnet.

Für einen Einsatz als Tensidalkohole spielt der Verzweigungsgrad der Olefine eine entscheidende Rolle. Der Verzweigungsgrad wird dabei beispielsweise durch den ISO-Index beschrieben, der die mittlere Zahl der Methylverzweigungen der jeweiligen Olefinfraktion angibt. So tragen z. B. bei einer $C_{12}$-Fraktion die n-Dodecene mit 0, Methylundecene mit 1 und Dimethyldecene mit 2 zum ISO-Index der Fraktion bei. Je niedriger der ISO-Index ist, umso größer ist die Linearität der Moleküle in der jeweiligen Fraktion.

Es ist bekannt, zur Herstellung wenig verzweigter Oligomere aus niederen Olefinen heterogene Katalysatoren einzusetzen, die als aktive Komponente überwiegend Nickel enthalten.

Beim DIMERSOL-Prozess (vgl. Revue de l'Institut Français du Petrole, Vol. 37, No. 5, Sept./Okt. 1982, S. 639ff) werden Propen oder Buten in homogener Phase in Gegenwart eines Katalysatorsystems aus einem Übergangsmetallderivat und einer metallorganischen Verbindung oligomerisiert. Typische Katalysatorsysteme sind Ni(O)-Komplexe in Verbindung mit Lewis-Säuren wie $AlCl_3$, $BF_3$, $SbF_5$ usw. oder Ni(II)-Komplexe in Verbindung mit Alkylaluminiumhalogeniden.

Heterogene Katalysatoren weisen gegenüber homogenen den Vorteil auf, dass eine Abtrennung des Katalysators vom Reaktoraustrag entfällt. Des Weiteren sind die Katalysatorkosten pro Tonne Produkt bei der homogenkatalysierten Fahrweise im Allgemeinen höher als bei einer heterogen-katalysierten Fahrweise.

Verfahren zur Oligomerisierung von Olefinen an Nickel enthaltenden heterogenen Katalysatoren zur Herstellung von Tensidalkoholen sind z. B. in der WO 00/53547, DE-A-198 59 911 und WO 00/56683 beschrieben.

Die WO 01/36356 beschreibt ein Verfahren zur Herstellung eines $C_{13}$-Alkoholgemischs, bei dem man

a) einen Butene enthaltenden $C_4$-Kohlenwasserstoffstrom, der weniger als 5 Gew.-%, bezogen auf die Butenfraktion iso-Buten enthält, bei erhöhter Temperatur mit einem Nickel enthaltenden heterogenen Katalysator in Kontakt bringt,

b) aus dem Reaktionsgemisch eine $C_{12}$-Olefinfraktion isoliert,

c) die $C_{12}$-Olefinfraktion durch Umsetzung mit Kohlenmonoxid und Wasserstoff in Gegenwart eines Kobalt-Katalysators hydroformyliert und

d) hydriert.

Dabei weist die $C_{12}$-Olefinfraktion einen ISO-Index von 1,9 bis 2,3 auf. Dieses Dokument beschreibt weiterhin die Verwendung der $C_{13}$-Alkoholgemische als Tensidalkohole.

Es ist bekannt, für die Oligomerisierung von Olefinen Nickel-haltige Katalysatoren auf Basis von Zeolithen des Faujasit-Typs einzusetzen. Derartige Katalysatoren sind in der US 3,402,217 und der EP-A-0 329 305 beschrieben. Es ist weiterhin bekannt, diese Nickel-haltigen Zeolithe einer Modifizierung zu unterziehen, um den Anteil an wenig verzweigten bzw. linearen Olefinen in dem bei der Oligomerisierung erhaltenen Olefingemisch zu erhöhen. Dazu zählt beispielsweise die Dotierung der Zeolithe mit Ca-, Cd-, Zn- oder Mn-Ionen.

Die US 4,029,719 beschreibt ein Verfahren zur Herstellung linearer Olefine durch Oligomerisierung von $C_3$-$C_{12}$-Olefinen unter Einsatz eines zeolithischen Katalysators, der durch Ionenaustausch mit Metallen der VIII. Nebengruppe beladen und zur Aktivierung mit einer organischen oder anorganischen Base behandelt wurde.

Die CA 1,205,792 beschreibt einen Katalysator für die Olefindimerisierung der durch Abscheiden von Nickel auf einem Zeolith und anschließendes Inkontaktbringen mit einem Amin erhalten wurde.

Die US 2004/0220440 A1 beschreibt ein Verfahren zur Oligomerisierung von olefinischen Kohlenwasserstoffströmen, die schwefelhaltige Verunreinigungen enthalten, bei dem man das Kohlenwasserstoffeinsatzmaterial zunächst mit einem ersten Metalloxidkatalysator zur Umwandlung der als Katalysatorgift wirkenden Schwefelverbindungen in unschädliche Verbindungen und dann mit einem zweiten Olefinoligomerisierungskatalysator in Kontakt bringt. Als erster Metalloxidkatalysator wird dabei vorzugsweise ein Nickel-haltiger Katalysator eingesetzt. Als möglicher zweiter Olefinoligomerisierungskatalysator wird eine Vielzahl verschiedener Zeolithe angegeben. Zum Olefineinsatzmaterial wird ganz allgemein und ohne Stützung durch ein Ausführungsbeispiel ausgeführt, dass es auch aus einem Oligomer bestehen oder ein solches Oligomer, z. B. aus der Rückführung eines Teils des Produktstroms, enthalten kann. Die Ausführungsbeispiele sind auf die Oligomerisierung von propenreichen $C_3$-Strömen beschränkt, die zuvor mit einem Nickel-haltigen Katalysator zur Umwandlung der enthaltenen Schwefelverbindungen in Kontakt gebracht wurden.

Die WO 2004/080935 beschreibt ein Verfahren zur Dimerisierung niederer olefinischer Kohlenwasserstoffe, bei dem man einen Olefin-Feed in einer Reaktionszone mit einem sauren Katalysator auf Basis eines natürlichen oder synthetischen Zeoliths mittlerer Porengröße in Kontakt bringt, einen dimerisierte Olefine enthaltenden Strom aus der Reaktionszone entnimmt, vom Austrag aus der Reaktionszone einen Dimerisierungsprodukte enthaltenden Strom und einen nicht umgesetzte Kohlenwasserstoffe enthaltenden Strom abtrennt und letzteren zumindest teilweise in die Reaktionszone zurückführt. Die Dimerisierung von Gemischen aus Olefinen unterschiedlicher Kohlenstoffatomanzahl bzw. eine Teilrückführung von Dimerisierungsprodukt wird nicht gelehrt.

Die WO 03/082780 beschreibt die Oligomerisierung von Olefin-Einsatzstoffen, die wenigstens ein Olefin mit 4 oder 5 Kohlenstoffatomen enthalten, in Gegenwart kristalliner Zeolithe als Oligomerisierungskatalysatoren, wobei ein Teil der erhaltenen Olefin-Oligomere in den Reaktor zurückgeführt wird.

Die WO 01/83407 beschreibt ein Verfahren zur Oligomerisierung von Alkenen mit 3 bis 6 Kohlenstoffatomen, wobei das Einsatzmaterial neben Alkenen mit x-Kohlenstoffatomen zusätzlich davon verschiedene Alkene mit y-Kohlenstoffatomen enthalten kann, wobei x und y unterschiedliche Bedeutungen haben, und die Oligomerisierung in Gegenwart eines zeolithischen Katalysators vom MFS-Strukturtyp erfolgt.

Die WO 00/56683 beschreibt ein Verfahren zur Herstellung von Tensid-Alkoholen, bei dem man ein Olefingemisch, das lineare Hexene enthält, zu einem Gemisch verzweigter Dodecene dimerisiert, anschließend zu Tensidalkoholen derivatisiert und gegebenenfalls alkoxiliert.

Die WO 01/36356 beschreibt die Herstellung eines $C_{13}$-Alkoholgemischs, bei dem man a) einen Butene enthaltenden $C_4$-Kohlenwasserstoffstrom, der w niger als 5 Gew.-%, bezogen auf die Butenfraktion, iso-Buten enthält, bei erhöhter Temperatur mit einem Nickel enthaltenden heterogenen Katalysator in Kontakt bringt, b) aus dem Reaktionsgemisch eine $C_{12}$-Olefinfraktion isoliert, c) die $C_{12}$-Olefinfraktion durch Umsetzung mit Kohlenmonoxid und Wasserstoff in Gegenwart eines Kobalt-Katalysators hydroformyliert und d) hydriert.

Die US 5,118,901 beschreibt ein Verfahren zur Herstellung höherer alpha-Olefine durch Umsetzung eines Olefingemischs, das ein erstes alpha-Olefin mit 2 bis 10 Kohlenstoffatomen und ein zweites alpha-Olefin mit 4 bis 10 Kohlenstoffatomen enthält, in Gegenwart eines Katalysators, der ein elementares Alkalimetall und ein Metalloxid enthält.

Die nicht vorveröffentlichte WO 2007/040812 beschreibt ein Verfahren zur Herstellung höherer Olefine, bei dem man ein Einsatzmaterial, das wenigstens ein $C_3$-$C_5$-Olefin und ein Dimer davon enthält einer Oligomerisierung in Gegenwart eines zeolithischen Oligomerisierungskatalysators unterzieht.

Werden zur Oligomerisierung Olefingemische eingesetzt, die Olefine unterschiedlicher Kohlenstoffatomzahl enthalten, so reagieren in der Regel bevorzugt zunächst die reaktiveren kürzerkettigen Olefine unter Dimerisierung zum Homo-Dimer. Bei diskontinuierlicher Fahrweise kann es dann zu einer starken Verarmung des Reaktionsgemischs an dem kürzerkettigen Olefin bis hin zu einer vollständigen Umsetzung des kürzerkettigen Olefins unter Bildung des Homo-Dimeren kommen, bevor auch das längerkettige Olefin einer Oligomerisierung unterzogen wird. Je nach Reaktivität des längerkettigen Olefins und der Homo-Dimeren oder -Oligomeren des kürzerkettigen Olefins werden dann bevorzugt Homo-Dimere des längerkettigen Olefins oder andere Oligomerisierungsprodukte, z. B. Co-Oligomere aus der Reaktion des längerkettigen Olefins mit Homo-Dimeren des kürzerkettigen Olefins, gebildet. Bei kontinuierlicher Fahrweise kann es im Extremfall zu einer ausschließlichen Bildung von Homo-Dimeren des kürzerkettigen Olefins kommen, wohingegen das nicht umgesetzte längerkettige Olefin sich im Wesentlichen vollständig im Reaktionsaustrag wieder findet. Zudem

wurden Co-Oligomerisierungsprodukte in der Vergangenheit vielfach als unerwünschte Nebenprodukte der Olefinoligomerisierung angesehen.

Überraschenderweise wurde nun gefunden, dass mit dem Einsatz von heterogenen Olefin-Oligomerisierungskatalysatoren eine Oligomerisierung von Olefingemischen unter Erzielung des Co-Oligomerisierungsprodukts in guter Ausbeute und Selektivität möglich ist. Dies gilt insbesondere für eine Oligomerisierung von Olefingemischen, die $C_n$-Olefine und $C_{2n}$-Olefine enthalten, wobei eine Erzielung des $C_{3n}$-Co-Oligomerisierungsprodukts in guter Ausbeute und Selektivität möglich ist. Weiterhin wurde überraschenderweise gefunden, dass sich für diese Co-Oligomerisierungen speziell heterogene Olefin-Oligomerisierungskatalysatoren auf Basis von Schicht- und/oder Gerüstsilicaten eignen. Überraschenderweise wurde weiterhin gefunden, dass die nach diesem Verfahren erhältlichen $C_{m+n}$-Co-Oligomerisierungsprodukte und speziell die $C_{3n}$-Co-Oligomerisierungsprodukte besonders vorteilhafte anwendungstechnische Eigenschaften, insbesondere bei einer Weiterverarbeitung zu Tensidalkoholen, aufweisen.

Gegenstand der Erfindung ist daher ein Verfahren zur Co-Dimerisierung von Olefinen, bei dem man

a) ein erstes Olefin-Einsatzmaterial bereitstellt, das im Wesentlichen aus $C_n$-Olefinen besteht und ein zweites Olefin-Einsatzmaterial durch Dimerisierung eines Raffinats II in Gegenwart eines nickelhaltigen Oligomerisierungskatalysators bereitstellt, das im Wesentlichen aus $C_m$-Olefinen besteht, wobei n und m unabhängig voneinander für zwei voneinander verschiedene ganze Zahlen von 2 bis 12 stehen, wobei das zweite Olefin-Einsatzmaterial einen Verzweigungsgrad der Olefine, bestimmt als ISO-Index, in einem Bereich von 0 bis 1,8 aufweist und
b) das erste und das zweite Olefin-Einsatzmaterial an einem heterogenen Olefin-Oligomerisierungskatalysator umsetzt, der einen Nickelgehalt, bezogen auf elementares Nickel, von höchstens 1 Gew.-% aufweist und der wenigstens ein Zeolith umfasst oder aus einem Zeolith besteht.

Im Rahmen der vorliegenden Erfindung umfasst der Begriff "Dimere" die Produkte aus der Vereinigung von zwei Olefinmolekülen zu einem Molekül, dessen Kohlenstoffatomanzahl der Summe der Kohlenstoffatome der zwei vereinigten Olefine entspricht. Die Dimere sind ihrerseits olefinisch ungesättigt. "Homo-Dimere" bezeichnet Dimere aus der Vereinigung von zwei identischen Olefinen. Enthält wenigstens eines der Olefin-Einsatzmaterialien ein Gemisch aus Olefinen gleicher Kohlenstoffatomanzahl, so bezeichnet "Homo-Dimere" Dimere aus der Vereinigung von zwei Olefinen gleicher Kohlenstoffatomanzahl. "Co-Dimere" bezeichnet Dimere aus der Vereinigung von Olefinen unterschiedlicher Kohlenstoffatomanzahl. Enthält wenigstens eines der Olefin-Einsatzmaterialien ein Gemisch aus Olefinen gleicher Kohlenstoffatomanzahl, so fallen auch die Co-Dimere in der Regel in Form eines Co-Dimerengemischs an.

Das erfindungsgemäße Verfahren ermöglicht die Herstellung eines Olefin-Oligomerisierungsprodukts aus einem ersten Olefin-Einsatzmaterial, das wenigstens ein $C_m$-Olefin enthält, und einem zweiten Olefin-Einsatzmaterial, das wenigstens ein $C_n$-Olefin enthält, wobei das Reaktionsprodukt einen wesentlichen Anteil an Co-Dimeren aus $C_m$-Olefinen und $C_n$-Olefinen (d. h. $C_{n+m}$-Olefinen) enthält. Unter M/46348-EP einem "wesentlichen Anteil" wird im Rahmen der vorliegenden Erfindung ein Anteil von wenigstens 5 Gew.-%, besonders bevorzugt wenigstens 10 Gew.-%, insbesondere wenigstens 12 Gew.-%, bezogen auf die Gesamtmenge der im Reaktionsaustrag enthaltenen Olefine (nicht umgesetzte Olefine, Homo-Dimere, Co-Dimere und davon verschiedene Oligomerisierungsprodukte) verstanden.

Die erfindungsgemäß eingesetzten Olefin-Oligomerisierungskatalysatoren ermöglichen eine Olefin-Co-Dimerisierung. Die Steuerung des Co-Dimerengehalts im Umsetzungsprodukt kann neben dem eingesetzten Katalysator (und neben weiteren Betriebsparametern, wie dem Druck und der Temperatur bei der Oligomerisierung und der Verweilzeit) über das Verhältnis von zugeführtem $C_m$-Olefin zu $C_n$-Olefin und, falls vorhanden, zu zurückgeführtem Material aus dem Umsetzungsprodukt gesteuert werden. Weitere wichtige Reaktionsparameter sind die Verweilzeit und der Umsatz.

In einer bevorzugten Ausführung wird in Schritt a) ein erstes Olefin-Einsatzmaterial bereitgestellt, das wenigstens ein Olefin mit zwei bis sechs Kohlenstoffatomen enthält ($C_n$-Olefin) und ein zweites Olefin-Einsatzmaterial, das wenigstens ein Olefin mit einer doppelt so großen Kohlenstoffatomanzahl wie das erste Olefin-Einsatzmaterial enthält ($C_{2n}$-Olefin). Das Reaktionsprodukt enthält dann einen wesentlichen Anteil an Co-Dimeren aus $C_n$-Olefinen und $C_{2n}$-Olefinen (d. h. $C_{3n}$-Olefinen).

Bei den in Schritt a) bereitgestellten Olefin-Einsatzmaterialien steht n vorzugsweise für 4, 5 oder 6, insbesondere für 4. Bei den in Schritt a) bereitgestellten Olefin-Einsatzmaterialien steht m vorzugsweise für 6 bis 10, insbesondere für 8.

Bevorzugte erste Olefin-Einsatzmaterialien für Schritt a) sind prinzipiell alle Verbindungen, welche 2 bis 6 Kohlenstoffatome und wenigstens eine ethylenisch ungesättigte Doppelbindung enthalten. Bevorzugt sind erste Olefin-Einsatzmaterialien, die Olefine mit 4 bis 6 Kohlenstoffatomen, insbesondere mit 4 Kohlenstoffatomen, enthalten. Bevorzugt sind die zur Oligomerisierung eingesetzten Olefine ausgewählt unter linearen (geradkettigen) Olefinen und Olefingemischen, die wenigstens ein lineares Olefin enthalten. Dazu zählen Ethen, Propen, 1-Buten, 2-Buten, 1-Penten, 2-Penten, 1-Hexen, 2-Hexen, 3-Hexen und Mischungen davon.

Vorzugsweise weist das erste Olefin-Einsatzmaterial einen Anteil an Olefinen mit der gleichen Kohlenstoffanzahl von wenigstens 70 Gew.-%, besonders bevorzugt wenigstens 90 Gew.-%, insbesondere 95 Gew.-% und speziell wenigstens 99 Gew.-%, bezogen auf den Gesamtolefingehalt, auf.

Vorzugsweise weist das erste Olefin-Einsatzmaterial einen Anteil an linearen Olefinen von wenigstens 30 Gew.-%, besonders bevorzugt wenigstens 40 Gew.-%, insbesondere wenigstens 50 Gew.-%, bezogen auf den Gesamtolefingehalt, auf.

In einer speziellen Ausführungsform wird ein erstes Olefin-Einsatzmaterial eingesetzt, das einen Anteil an verzweigten Olefinen von höchstens 20 Gew.-%, besonders bevorzugt von höchstens 10 Gew.-%, insbesondere von höchstens 5 Gew.-%, speziell von höchstens 3 Gew.-%, bezogen auf den Gesamtolefingehalt, aufweist.

Vorzugsweise wird in Schritt a) des erfindungsgemäßen Oligomerisierungsverfahrens ein technisch zur Verfügung stehendes olefinhaltiges Kohlenwasserstoffgemisch als erstes Olefin-Einsatzmaterial eingesetzt.

Bevorzugte großtechnisch zur Verfügung stehende Olefingemische resultieren aus der Kohlenwasserstoff-Spaltung bei der Erdölverarbeitung, beispielsweise durch Katcracken, wie Fluid Catalytic Cracking (FCC), Thermocracken oder Hydrocracken mit anschließender Dehydrierung. Ein geeignetes technisches erstes Olefingemisch ist der $C_4$-Schnitt. $C_4$-Schnitte sind beispielsweise durch Fluid Catalytic Cracking oder Steamcracken von Gasöl bzw. durch Steamcracken von Naphtha erhältlich. Je nach Zusammensetzung des $C_4$-Schnitts unterscheidet man den Gesamt-$C_4$-Schnitt (Roh-$C_4$-Schnitt), das nach der Abtrennung von 1,3-Butadien erhaltene so genannte Raffinat I sowie das nach der Isobutenabtrennung erhaltene Raffinat II. Ein weiteres geeignetes technisches erstes Olefingemisch ist der bei der Naphtha-Spaltung erhältliche $C_5$-Schnitt. Für den Einsatz in Schritt a) geeignete olefinhaltige Kohlenwasserstoffgemische mit 4 bis 6 Kohlenstoffatomen lassen sich weiterhin durch katalytische Dehydrierung geeigneter großtechnisch zur Verfügung stehender Paraffingemische erhalten. So gelingt beispielsweise die Herstellung von $C_4$-Olefin-Gemischen aus Flüssiggasen (liquified petroleum gas, LPG) und verflüssigbaren Erdgasen (liquified natural gas, LNG). Letztere umfassen neben der LPG-Fraktion auch zusätzlich größere Mengen höhermolekularer Kohlenwasserstoffe (leichtes Naphtha) und eignen sich somit auch zur Herstellung von $C_5$- und $C_6$-Olefin-Gemischen. Die Herstellung von olefinhaltigen Kohlenwasserstoffgemischen, die Monoolefine mit 4 bis 6 Kohlenstoffatomen enthalten, aus LPG- oder LNG-Strömen gelingt nach üblichen, dem Fachmann bekannten Verfahren, die neben der Dehydrierung in der Regel noch einen oder mehrere Aufarbeitungsschritte umfassen. Dazu zählt beispielsweise die Abtrennung wenigstens eines Teils der in den zuvor genannten Olefin-Einsatzgemischen enthaltenen gesättigten Kohlenwasserstoffe. Diese können beispielsweise erneut zur Herstellung von Olefin-Einsatzmaterialien durch Crackung und/oder Dehydrierung eingesetzt werden. Die in dem erfindungsgemäßen Verfahren eingesetzten Olefine können jedoch auch einen Anteil gesättigter Kohlenwasserstoffe enthalten, die sich gegenüber den erfindungsgemäßen Oligomerisierungsbedingungen inert verhalten. Der Anteil dieser gesättigten Komponenten beträgt im Allgemeinen höchstens 60 Gew.-%, bevorzugt höchstens 40 Gew.-%, besonders bevorzugt höchstens 20 Gew.-%, bezogen auf die Gesamtmenge der in dem Kohlenwasserstoff-Einsatzmaterial enthaltenen Olefine und gesättigten Kohlenwasserstoffe.

Ein zum Einsatz in dem erfindungsgemäßen Verfahren geeignetes Raffinat II hat beispielsweise die folgende Zusammensetzung:

0,5 bis 5 Gew.-% Isobutan,
5 bis 30 Gew.-% n-Butan,
20 bis 40 Gew.-% trans-2-Buten,
10 bis 20 Gew.-% cis-2-Buten,
25 bis 55 Gew.-% 1-Buten,
0,5 bis 5 Gew.-% Isobuten

sowie Spurengase, wie 1,3-Butadien, Propen, Propan, Cyclopropan, Propadien, Methylcyclopropan, Vinylacetylen, Pentene, Pentane etc. im Bereich von jeweils maximal 1 Gew.-%.

Ein geeignetes Raffinat II weist folgende typische Zusammensetzung auf:

| | |
|---|---|
| Butane | 26 Gew.-% |
| i-Buten | 1 Gew.-% |
| 1-Buten | 26 Gew.-% |
| trans-2-Buten | 31 Gew.-% |
| cis-2-Buten | 16 Gew.-% |

Sind Diolefine oder Alkine im ersten olefinreichen Kohlenwasserstoffgemisch vorhanden, so können diese vor der Oligomerisierung auf vorzugsweise weniger als 200 Gew.-ppm aus demselben entfernt werden. Sie werden bevorzugt durch selektive Hydrierung, z. B. gemäß EP-81 041 und DE-15 68 542 entfernt, besonders bevorzugt durch eine selektive Hydrierung bis auf einen Restgehalt von unter 100 Gew.-ppm, insbesondere 10 Gew.-ppm.

Aus dem olefinreichen Kohlenwasserstoffgemisch werden zweckmäßigerweise außerdem sauerstoffhaltige

Verbindungen, wie Alkohole, Aldehyde, Ketone oder Ether, weitgehend entfernt. Hierzu kann das olefinreiche Kohlenwasserstoffgemisch mit Vorteil über ein Adsorptionsmittel, wie z. B. ein Molekularsieb, bevorzugt ein Adsorptionsmittel wie in der DE-A-19845857 beschrieben, worauf diesbezüglich Bezug genommen wird, geleitet werden. Die Konzentration an sauerstoffhaltigen, schwefelhaltigen, stickstoffhaltigen und halogenhaltigen Verbindungen im olefinreichen Kohlenwasserstoffgemisch beträgt vorzugsweise weniger als 20 Gew.-ppm, besonders bevorzugt weniger als 10 Gew.-ppm, insbesondere weniger als 1 Gew.-ppm.

Bevorzugte zweite Olefin-Einsatzmaterialien für Schritt a) sind prinzipiell alle Verbindungen, welche 4 bis 12 Kohlenstoffatome und eine ethylenisch ungesättigte Doppelbindung enthalten. Bevorzugt sind zweite Olefin-Einsatzmaterialien, die Olefine mit 8 bis 12 Kohlenstoffatomen, insbesondere Olefine mit 8 Kohlenstoffatomen, enthalten. Bevorzugt sind die zur Oligomerisierung eingesetzten Olefine ausgewählt unter linearen und wenig verzweigten Olefinen und Olefingemischen.

Geeignete Octene sind z. B. 1-Octen, 2-Octen, 3-Octen, 4-Octen, 2-Methyl-hept-1-en, 2-Methyl-hept-2-en, 2-Methyl-hept-3-en, 6-Methyl-hept-3-en, 6-Methyl-hept-2-en, 6-Methyl-hept-1-en, 3-Methyl-hept-1-en, 3-Methyl-hept-2-en, 3-Methyl-hept-3-en, 5-Methyl-hept-3-en, 5-Methyl-hept-2-en, 5-Methyl-hept-1-en, 4-Methyl-hept-1-en, 4-Methyl-hept-2-en, 4-Methyl-hept-3-en, und Mischungen davon.

Vorzugsweise weist das zweite Olefin-Einsatzmaterial einen Anteil an Olefinen mit der gleichen Kohlenstoffatomanzahl von wenigstens 70 Gew.-%, besonders bevorzugt wenigstens 90 Gew.-%, insbesondere wenigstens 95 Gew.-%, speziell wenigstens 99 Gew.-%, auf.

Das zweite Olefin-Einsatzmaterial weist einen Verzweigungsgrad der Olefine, bestimmt als ISO-Index, in einem Bereich von 0 bis 1,8, besonders bevorzugt von 0,5 bis 1,5, insbesondere von 0,8 bis 1,3, auf.

Vorzugsweise wird in Schritt a) des erfindungsgemäßen Oligomerisierungsverfahrens ein technisch zur Verfügung stehendes zweites Olefin-Einsatzmaterial eingesetzt.

Erfindungsgemäß wird das zweite Olefin-Einsatzmaterial durch Dimerisierung eines Raffinats II, wie zuvor definiert, in Gegenwart eines Nickel-haltigen Oligomerisierungskatalysators erhalten.

Die Umsetzung der Olefin-Einsatzmaterialien in Schritt b) erfolgt vorzugsweise kontinuierlich.

Dazu wird in ein Reaktorsystem das erste Olefin-Einsatzmaterial und das zweite Olefin-Einsatzmaterial eingespeist und an dem Olefin-Oligomerisierungskatalysator umgesetzt.

In einer speziellen Ausführungsform wird das Umsetzungsprodukt aus Schritt b) in einen ersten und einen zweiten Teilstrom aufgetrennt, der erste Teilstrom einer Aufarbeitung unter Erhalt einer im Wesentlichen das Co-Dimerisierungsprodukt enthaltenden Fraktion unterzogen und der zweite Teilstrom in Schritt a) zurückgeführt. Dieser zurückgeführte Zufuhrstrom besteht im Wesentlichen aus Oligomeren, nicht umgesetzten Olefinen und gegebenenfalls gesättigten Kohlenwasserstoffen.

In einer speziellen Ausführung wird in das Reaktionssystem zusätzlich ein bei der Aufarbeitung des Umsetzungsprodukts aus Schritt b) bzw. des ersten Teilstroms des Umsetzungsprodukts aus Schritt b) gewonnener olefinhaltiger Strom eingespeist. das molare Verhältnis von $C_m$-Olefinen zu $C_n$-Olefinen, insbesondere das molare Verhältnis von $C_{2n}$-Olefinen zu $C_n$-Olefinen, bezogen auf die Gesamtolefinmenge der zugeführten liegt vorzugsweise in einem Bereich von 0,25 : 1 bis 4 : 1, besonders bevorzugt in einem Bereich von 0,5 : 1 bis 3 : 1, insbesondere in einem Bereich von 1 : 1 bis 2,5 : 1.

Das in Schritt b) des erfindungsgemäßen Verfahrens eingesetzte Reaktionssystem kann einen oder mehrere, gleiche oder verschiedene Reaktoren umfassen. Im einfachsten Fall wird das Reaktionssystem von einem einzelnen Reaktor gebildet. Werden mehrere Reaktoren eingesetzt, so können diese jeweils gleiche oder verschiedene Vermischungscharakteristiken aufweisen. Die einzelnen Reaktoren können gewünschtenfalls durch Einbauten ein- oder mehrfach unterteilt sein. Bilden zwei oder mehrere Reaktoren das Reaktionssystem, so können diese untereinander beliebig verschaltet sein, z. B. parallel oder in Reihe. In einer bevorzugten Ausführung wird ein Reaktionssystem eingesetzt, das aus zwei in Reihe geschalteten Reaktoren besteht.

Geeignete druckfeste Reaktionsapparaturen für die Oligomerisierung sind dem Fachmann bekannt. Dazu zählen die allgemein üblichen Reaktoren für Gas-fest- und Gas-flüssig-Reaktionen, wie z. B. Rohrreaktoren, Rührkessel, Gasumlaufreaktoren, Blasensäulen etc., die gegebenenfalls durch Einbauten unterteilt sein können. Vorzugsweise werden Rohrbündelreaktoren oder Schachtöfen eingesetzt. In dem Reaktor oder den Reaktoren kann der Katalysator in einem einzigen oder in mehreren Katalysator-Festbetten angeordnet sein. Dabei ist es möglich, in den einzelnen Reaktionszonen unterschiedliche Katalysatoren einzusetzen. Bevorzugt ist jedoch der Einsatz des gleichen Katalysators in allen Reaktionszonen.

Die Temperatur bei der Oligomerisierungsreaktion liegt im Allgemeinen in einem Bereich von etwa 20 bis 280 °C, bevorzugt von 25 bis 200 °C, insbesondere von 30 bis 140 °C. Umfasst das Reaktionssystem mehr als einen Reaktor, so können diese gleiche oder verschiedene Temperaturen aufweisen. Des Gleichen kann ein Reaktor mehrere Reaktionszonen aufweisen, die bei verschiedenen Temperaturen betrieben werden. So kann beispielsweise in einer zweiten Reaktionszone eines einzelnen Reaktors eine höhere Temperatur als in der ersten Reaktionszone oder im zweiten Reaktor einer Reaktorkaskade eine höhere Temperatur als im ersten Reaktor eingestellt werden, z. B. um einen möglichst

vollständigen Umsatz zu erzielen.

Der Druck bei der Oligomerisierung liegt im Allgemeinen in einem Bereich von etwa 1 bis 300 bar, vorzugsweise von 5 bis 100 bar und insbesondere von 10 bis 70 bar. Der Reaktionsdruck kann beim Einsatz mehrerer Reaktoren in den einzelnen Reaktoren unterschiedlich sein.

In einer speziellen Ausführung werden die zur Oligomerisierung eingesetzten Temperatur und Druckwerte so gewählt, dass das olefinhaltige Einsatzmaterial flüssig oder im überkritischen Zustand vorliegt.

Die Umsetzung in Schritt b) erfolgt vorzugsweise nicht adiabatisch (sondern vorzugsweise unter Abführung der Reaktionswärme durch Wärmetausch mit einem externen Wärmeträgermedium). Geeignete Vorrichtungen zum Wärmetausch und zur Abführung von Prozesswärme sind die üblichen, dem Fachmann bekannten. Die Wärmeaustauschvorrichtung kann an bzw. im Reaktor angebracht sein. Nach der zuvor beschriebenen Verfahrensvariante mit Rückführung eines zweiten Teilstroms des Umsetzungsprodukts wird dem Teilstrom Wärme durch Inkontaktbringen mit einem externen Wärmeträgermedium entzogen. Die gewonnene Wärmemenge kann an anderer Stelle des Verfahrens, z. B. bei der Auftrennung des Umsetzungsprodukts, wieder eingesetzt werden.

Gewünschtenfalls kann die Umsetzung in Schritt b) auch adiabatisch durchgeführt werden. Dieser Begriff wird im Rahmen der vorliegenden Erfindung im technischen und nicht im physiko-chemischen Sinne verstanden. So verläuft die Oligomerisierungsreaktion in der Regel exotherm, so dass das Reaktionsgemisch beim Strömen durch das Reaktionssystem, beispielsweise ein Katalysatorbett, eine Temperaturerhöhung erfährt. Unter adiabatischer Reaktionsführung wird eine Vorgehensweise verstanden, bei der die in einer exothermen Reaktion freiwerdende Wärmemenge von der Reaktionsmischung im Reaktor aufgenommen und keine Kühlung durch Kühlvorrichtungen angewandt wird. Somit wird die Reaktionswärme mit dem Reaktionsgemisch aus dem Reaktor abgeführt, abgesehen von einem Restanteil, der durch natürliche Wärmeleitung und Wärmeabstrahlung vom Reaktor an die Umgebung abgegeben wird.

Der in Schritt b) eingesetzte Olefin-Oligomerisierungskatalysator umfasst vorzugsweise wenigstens ein Silicat, das ausgewählt ist unter Schichtsilicaten, Gerüstsilicaten und Kombinationen davon.

Der erfindungsgemäß in Schritt b) eingesetzte Olefin-Oligomerisierungskatalysator enthält im Wesentlichen kein Nickel. Im Sinne der Erfindung wird darunter ein Katalysator verstanden, der einen Nickelgehalt, bezogen auf elementares Nickel, von höchstens 1 Gew.-%, besonders bevorzugt von höchstens 0,1 Gew.-%, insbesondere von höchstens 0,01 Gew.-%, bezogen auf das Gesamtgewicht des Katalysators, aufweist.

Es wurde gefunden, dass Olefin-Oligomerisierungskatalysatoren mit hoher Kristallinität der in ihnen als Aktivkomponente enthaltenen Silicate besonders vorteilhafte Eigenschaften bei der Co-Oligomerisierung von Olefinströmen aufweisen. Im Sinne der Erfindung weisen die einzelnen Atome der kristallinen Schichtsilicate oder Gerüstsilicate eine regelmäßige Fernordnung in einer Gitterstruktur auf. Kristalline Silicate weisen bei der Röntgendiffraktometrie (XRD) charakteristische Bandenlagen auf. Der kristalline Anteil lässt sich über die Intensität der Reflexe, bzw. ihre Breite, quantitativ bestimmen. Bevorzugt weisen die Schicht- und/oder Gerüstsilicate des in Schritt b) eingesetzten Oligomerisierungskatalysators einen kristallinen Anteil von wenigstens 50 Gew.-%, besonders bevorzugt von wenigstens 75 Gew.-%, auf.

Der in Schritt b) eingesetzte Olefin-Oligomerisierungskatalysator umfasst vorzugsweise wenigstens ein mikro- oder mesoporöses Silicat. Als mikroporös werden nach IUPAC Feststoffe mit einem mittleren Porenradius von weniger als 2 nm bezeichnet. Als mesoporös werden Feststoffe mit einem mittleren Porenradius im Bereich von 2 bis 50 nm bezeichnet.

Erfindungsgemäß umfasst der in Schritt b) eingesetzte Olefin-Oligomerisierungskatalysator wenigstens ein Zeolith oder besteht aus wenigstens einem Zeolith.

Als Ausgangsmaterial für die erfindungsgemäß eingesetzten Katalysatoren geeignete Zeolithe sind prinzipiell die unter dieser Bezeichnung bekannten kristallinen, natürlich vorkommenden oder synthetischen Gerüstsilicate. Diese können in ihrer Zusammensetzung variieren, weisen jedoch im Allgemeinen neben Silicium, Aluminium und Sauerstoff wenigstens ein Alkali- und/oder Erdalkalimetall auf. Gewünschtenfalls kann bei den Zeolithen das Aluminium durch eines oder mehrere davon verschiedene Atome teilweise oder vollständig ersetzt werden. Die von Aluminium verschiedenen Atome sind vorzugsweise ausgewählt unter B, Ga, Fe, Si und Ti.

Bevorzugt werden in dem erfindungsgemäßen Verfahren Zeolithe mit einem mittleren Porendurchmesser von wenigstens 5 Å, besonders bevorzugt wenigstens 6 Å insbesondere wenigstens 7 Å eingesetzt.

Bevorzugt eingesetzte Zeolithe sind ausgewählt unter folgenden Strukturtypen: BEA, MFI, MEL, FAU, MOR, MWW, LTL, LTA, CHA, TON, MTW, FER, MAZ, EPI und GME. Besonders bevorzugt eingesetzte Zeolithe sind ausgewählt unter folgenden Strukturtypen: BEA, MFI, MEL, FAU, MOR, MWW, FER.

Die erfindungsgemäß als oder in den Olefin-Oligomerisierungskatalysatoren eingesetzten Silicate, speziell Zeolithe, können z. B. in der $H^+$, Ammonium-, Alkali- oder Erdalkali-Form eingesetzt werden.

Die erfindungsgemäß eingesetzten Silicate, speziell Zeolithe, können vor ihrem Einsatz zur Olefin-Oligomerisierung wenigstens einem Modifizierungsschritt unterzogen werden. Dazu zählt z. B. eine Modifizierung mit Säuren, Ammonium- und/oder mit Metallsalzlösungen. Dazu zählt weiterhin eine Dealuminierung des in des Silicatgerüsts eingebauten Aluminiums, Dehydroxylierung, Extraktion von "extra-framework" Aluminiumoxid oder Silylierung. Des Weiteren kann der Olefin-Oligomerisierungskatalysator zur Modifizierung einer Formgebung, thermischen Behandlung oder einer Behandlung mit Wasserdampf (steaming) unterzogen werden. Durch eine solche Modifizierung ist es möglich, eine möglichst hohe Selektivität, hohe Umsätze, lange Katalysator-Standzeiten und/oder eine hohe Anzahl möglicher Regenerationszyklen zu erreichen.

Eine Modifizierung der erfindungsgemäß eingesetzten Silicate kann in einer bevorzugten Ausführung durch inniges miteinander Inkontaktbringen mit wässrigen Salzlösungen erfolgen, die Ammoniumsalze, Salze von Alkalimetallen wie Na und K, Erdalkalimetallen wie Ca, Mg, Erdmetallen wie Tl, Übergangsmetallen wie z. B. Ti, Zr, Mn, Fe, Mo, Cu, Zn, Cr, Edelmetallen oder seltenen Erdmetallen wie z. B. La, Ce oder Y oder Mischungen davon enthalten. Dabei kommt es in der Regel zu einem zumindest teilweisen Ionenaustausch von Kationen des Silicats, speziell des Zeoliths, gegen andere Kationen aus der Salzlösung. Vorzugsweise erfolgt das Inkontaktbringen mit der Salzlösung so, dass das Silicat vollständig von Salzlösung umgeben ist. Dazu eignen sich beispielsweise die üblichen Tauch- und Tränkverfahren, wie sie zur Katalysatorherstellung bekannt sind. Bevorzugt wird die Salzlösung während der Behandlung des Silicats an diesem vorbei bewegt, z. B. durch Rühren oder Umpumpen.

In einer bevorzugten Ausführungsform wird in Schritt b) des erfindungsgemäßen Verfahrens ein saurer Olefin-Oligomerisierungskatalysator eingesetzt. Geeignete saure Zentren aufweisende Silicate können Lewis- und/oder Brönstedt-Säuren aufweisen. Es kann sich sowohl um natürlich vorkommende saure Silicate wie um Silicate handeln die durch Inkontaktbringen mit wenigstens einer Lewis- und/oder Brönstedt-Säure sauer modifiziert wurden. Schichtsilikate mit negativen Ladungen kommen in der Natur z. B. in Form von Montmorilloniten, Vermiculiten oder Hectoriten vor. Näheres zu sauren Schichtsilicaten ist Z. M. Thomas und W. Z. Thomas, Principles and Practice of Heterogeneous-Catalysis, 1997, Vetc. ISBN 3-527-29239-8, S. 347 ff. zu entnehmen.

Eine Modifizierung der erfindungsgemäß eingesetzten Silicate kann in einer bevorzugten Ausführung durch inniges miteinander Inkontaktbringen mit Lewis- oder Brönsted-Säuren, bevorzugt mit Protonensäuren und insbesondere mit wässrigen Lösungen wenigstens einer Protonensäure erfolgen. Dabei kommt es in der Regel zu einer teilweise oder vollständigen Protonenabsättigung der negativen Ladungen in den Silicaten durch teilweisen oder vollständigen Austausch der enthaltenen, austauschfähigen Kationen, in der Regel Alkali- oder Erdalkaliionen, gegen Protonen. Dies geschieht in an sich bekannter Weise, z. B. durch Behandlung mit Salpetersäure oder Salzsäure oder durch Behandlung mit Ammoniumsalzen und anschließendes Austreiben von Ammoniak durch Kalzination.

In einer bevorzugten Ausführungsform des erfindungsgemäßen Verfahrens wird in Schritt b) als Olefin-Oligomerisierungskatalysator ein Zeolith in der $H^+$-Form eingesetzt.

Die erfindungsgemäß einzusetzenden Silicate können geträgert und/oder einer Formgebung unterzogen werden.

Als Trägermaterial für die erfindungsgemäßen Katalysatoren können praktisch alle Trägermaterialien des Stands der Technik, wie sie vorteilhaft bei der Herstellung von geträgerten Katalysatoren Verwendung finden, beispielsweise $SiO_2$ (Quarz), Porzellan, Magnesiumoxid, Zinndioxid, Siliciumcarbid, Rutil, $Al_2O_3$ (Tonerde), Tone, Aluminiumsilikat, Steatit (Magnesiumsilicat), $ZrO_2$, Zirkoniumsilicat, Cersilicat, Cellulosematerialien, Polymere, Metalle, Graphit, oder Kombination aus wenigstens zwei dieser Trägermaterialien, eingesetzt werden. Weiterhin ist es möglich, die Katalysatoren auf Glas oder anderen Körpern wie z. B. Geweben (insbesondere Metallgeweben) jeglicher Art, speziell in Form von Monolithen, einzusetzen.

Die Olefin-Oligomerisierungskatalysatoren können in Form eines Pulvers eingesetzt werden. Vorzugsweise werden die Olefin-Oligomerisierungskatalysatoren in stückiger (teilchenförmiger) Form eingesetzt. Die Katalysatorteilchen weisen im Allgemeinen einen Mittelwert des (größten) Durchmessers von 0,5 bis 20 mm, vorzugsweise 1 bis 10 mm, auf. Dazu zählen z. B. Katalysatoren in Form von Granulat oder Splitt, z. B. mit einem Partikeldurchmesser von vorzugsweise 0,5 bis 5 mm, insbesondere von 0,5 bis 2 mm, Tabletten, z. B. mit einem Durchmesser von 2 bis 6 mm und einer Höhe von 3 bis 5 mm, Ringen mit z. B. 5 bis 7 mm Außendurchmesser, 2 bis 5 mm Höhe und 2 bis 3 mm Lochdurchmesser, oder Strängen unterschiedlicher Länge eines Durchmessers von z. B. 1,0 bis 5 mm. Derartige Formen können auf an sich bekannte Weise durch Tablettierung, Strangpressen oder Extrusion erhalten werden. Zur Herstellung von Granulat oder Splitt können größere Formkörper zerkleinert werden. Dieses Granulat oder dieser Splitt und auch die anderen genannten Katalysatorformkörper enthalten vorzugsweise praktisch keine feinkörnigeren Anteile als solche mit 0,5 mm Mindestpartikeldurchmesser.

Zur Formgebung durch Tablettierung, Strangpressen oder Extrusion können der Silicatmasse übliche Hilfsmittel zugesetzt werden. Dazu zählen Gleitmittel, Bindemittel, Formhilfsmittel und/oder Verstärkungsmittel. Geeignete Gleitmittel sind z. B. Graphit, Polyethylenoxid, Cellulose oder Fettsäuren (wie Stearinsäure). Geeignet Bindemittel, Formhilfsmittel und Verstärkungsmittel sind z. B. ausgewählt unter Aluminiumoxiden, bevorzugt Boehmit, Titandioxid, von den als katalytisch aktive Masse eingesetzten verschiedenen Silicaten, sowie Fasern aus Glas, Asbest oder Siliciumcarbid. Bevorzugte Bindemittel sind amorphe Alumosilicate, Siliciumdioxid, bevorzugt hochdisperses Siliciumdioxid, wie z. B. Silicasole, Gemische aus hochdispersem Siliciumdioxid und hochdispersem Aluminiumoxid, hochdisperses Titandioxid und Tone. Werden Bindemittel und/oder andere Hilfsmittel eingesetzt, ist der Extrusion bzw. der Tablettierung zweckmäßigerweise ein Mischungs- oder Knetprozess vorgeschaltet.

Gegebenenfalls erfolgt nach einer chemischen Modifizierung und/oder Trägerung/- Formgebung noch ein Kalzinierungsschritt.

Wurde der Katalysator einer nasschemischen Modifizierung unterzogen, so kann man ihn vor der Kalzinierung zusätzlich einer Trocknung unterziehen. Die Temperatur bei der Trocknung beträgt vorzugsweise 40 bis 180 °C, besonders bevorzugt 80 bis 150 °C. Die Trocknung kann in dazu üblichen Vorrichtungen, z. B. Bandtrocknern, Trockenschränken und Trockenkammern, erfolgen. Zur Trocknung kann zusätzlich ein Gasstrom (z. B. ein Luftstrom) an den Teilchen vorbeigeleitet werden.

Die Kalzinierung erfolgt vorzugsweise bei einer Temperatur im Bereich von etwa 200 bis 600 °C, besonders bevorzugt 300 bis 550 °C. Vorzugsweise erfolgt die Kalzinierung in einem Gasstrom, in der Regel einem Luftstrom. Die bei der Kalzinierung eingesetzte Gasmenge liegt (bezogen auf Katalysatormenge und die Zeit) beispielsweise in einem Bereich von etwa 100 bis 2000 l/l x h. Die Dauer der Kalzinierung beträgt vorzugsweise wenigstens 30 Minuten, besonders bevorzugt wenigstens eine Stunde. In der Regel ist eine Kalzinierungsdauer von höchstens 24 Stunden, besonders bevorzugt höchstens 12 Stunden, ausreichend. Zusätzlich zur Kalzinierung kann der erfindungsgemäße Katalysator vor einem Einsatz zur Oligomerisierung von Olefinen einer Aktivierung unterzogen werden. Vorzugsweise erfolgt die Aktivierung bei einer Temperatur im Bereich von etwa 150 bis 400 °C, besonders bevorzugt 200 bis 300 °C. Die Aktivierung erfolgt vorzugsweise im Gasstrom, besonders bevorzugt in Gegenwart von Sauerstoffabgereicherter Luft oder eines Inertgases. Die zur Aktivierung eingesetzte Gasmenge liegt vorzugsweise in einem Bereich von etwa 100 bis 2000 l/l x h. Die Aktivierungsdauer beträgt vorzugsweise wenigstens 30 Minuten, besonders bevorzugt wenigstens eine Stunde. In der Regel ist eine Aktivierungsdauer von höchstens 24 Stunden, besonders bevorzugt von höchstens 12 Stunden, ausreichend.

Durch Behandeln mit Wasserdampf kann die Art und der Mengenanteil der erhaltenen Oligomerisierungsprodukte optimiert werden. Dies gilt insbesondere für Katalysatoren auf Basis saurer Silicate, speziell saurer Zeolithe.

Die erfindungsgemäßen Katalysatoren weisen vorzugsweise 10 bis 100 Gew.-%, besonders bevorzugt 20 bis 95 Gew.-%, insbesondere 30 bis 80 Gew.-% Silicataktivmasse, bezogen auf ihr Gesamtgewicht, auf.

Die zuvor beschriebenen Katalysatoren zeichnen sich gegenüber entsprechenden Katalysatoren des Stands der Technik auf Basis von Nickel, speziell Nickeloxid, durch eine höhere Aktivität und/oder Selektivität bezüglich der Bildung von Co-Dimeren aus. Das in Schritt b) entnommene Umsetzungsprodukt weist vorzugsweise einen Gehalt an Co-Dimeren ($C_{m+n}$-Olefinen, bzw. $C_{3n}$-Olefinen) von wenigstens 5 Gew.-%, besonders bevorzugt von wenigstens 10 Gew.-%, insbesondere von wenigstens 12 Gew.-%, bezogen auf das Gesamtgewicht des entnommenen Umsetzungsprodukts, auf.

Das in Schritt b) entnommene Umsetzungsprodukt weist vorzugsweise einen Verhältnis von Homo-Dimeren des höheren Olefins (nach der speziellen Ausführung an $C_{4n}$-Olefinen, d.h. Homo-Dimeren des $C_{2n}$-Olefins) zu Co-Dimeren (speziell $C_{3n}$-Olefine) von 1 : ≥ 3, besonders bevorzugt von 1 : ≥ 4, insbesondere von 1 : ≥ 5, auf.

Das in Schritt b) erhaltene Umsetzungsprodukt (d. h. der Austrag aus dem Reaktionssystem) kann einer Aufarbeitung nach üblichen, dem Fachmann bekannten Verfahren unterzogen werden.

In einer speziellen Ausführung wird das Umsetzungsprodukt aus Schritt b) nach dem Austritt aus dem Reaktionssystem in einen ersten und einen zweiten Teilstrom aufgetrennt. Diese Auftrennung kann mit einer üblichen im Austrittsrohr angebrachten Trennvorrichtung erfolgen. Diese besteht im einfachsten Fall aus einer im Austrittsrohr befindlichen Trennwand geeigneter Geometrie (z. B. Y-Kupplung), von der ausgehend der Austrittsstrom in zwei Rohre für je einen der Teilströme überführt wird. Bei festen Mengenverhältnissen der Teilströme können diese über den Durchmesser der abzweigenden Rohre festgelegt werden. Zur Regelung der Mengenanteile der beiden Teilströme kann in einem oder in beiden der abzweigenden Rohre eine Schiebervorrichtung angebracht sein. Eine geregelte Entnahme der Teilströme ist alternativ auch über Pumpen möglich.

Vorzugsweise beträgt nach dieser Ausführungsform der Anteil des aufzuarbeitenden ersten Teilstroms 1 bis 50 Gew.-%, bevorzugt 2 bis 30 Gew.-%, insbesondere 5 bis 20 Gew.-%, bezogen auf das Gesamtgewicht des Austrags. Der Anteil des zweiten Teilstroms (Wälzstrom) beträgt dementsprechend bevorzugt 50 bis 99 Gew.-%, besonders bevorzugt 70 bis 98 Gew.-%, insbesondere 80 bis 95 Gew.-%, bezogen auf das Gesamtgewicht des Austrags.

Der zweite Teilstrom wird im Allgemeinen chemisch unverändert in das Reaktionssystem zurückgeführt. Gewünschtenfalls können die Temperatur und/oder der Druck vor der Zurückführung auf die gewünschten Werte eingestellt werden. Die Einspeisung des zweiten Teilstroms in das Reaktionssystem kann gemeinsam mit einem oder beiden Olefin-Einsatzmaterialien oder separat davon erfolgen. Das Gewichtsmengenverhältnis von in das Reaktionssystem eingespeistem zweiten Teilstrom zu frischem olefinhaltigen Zulauf insgesamt liegt vorzugsweise in einem Bereich von 1 : 1 bis 50 : 1, besonders bevorzugt von 2 : 1 bis 30 : 1, insbesondere von 5 : 1 bis 20 : 1.

Das Umsetzungsprodukt aus Schritt b) bzw. der erste Teilstrom des Austrags aus dem Reaktionssystem wird einer Aufarbeitung nach üblichen, dem Fachmann bekannten Verfahren unterzogen. Der Austrag bzw. der erste Teilstrom wird dazu einer ein- oder mehrstufigen Trennoperation unterzogen, wobei zumindest ein die Hauptmenge des Co-Dimerisierungsprodukts enthaltender Strom und ein im Wesentlichen aus nicht umgesetztem Olefin und gegebenenfalls gesättigtem Kohlenwasserstoff bestehender Strom erhalten werden. Gesättigte Kohlenwasserstoffe stammen beispielsweise aus den zur Oligomerisierung eingesetzten olefinhaltigen Zuläufen, die diese als Beimischung enthalten können, oder beispielsweise zu einem geringen Ausmaß aus einer Partialhydrierung der eingesetzten Olefine zur Entfernung von Diolefinen. In Abhängigkeit von den angewandten Trennverfahren werden gegebenenfalls weitere Ströme erhalten, wie spezielle Oligomerfraktionen oder im Wesentlichen aus gesättigten Kohlenwasserstoffen bestehende Ströme.

Vorzugsweise wird als Austrag aus dem Reaktionssystem ein flüssiger Strom entnommen und zur Aufarbeitung zumindest teilweise in die Gasphase überführt. In der einfachsten Ausführung wird dazu der flüssige Reaktoraustrag einer Erwärmung und/oder Entspannung unterzogen, wobei eine Auftrennung in eine Flüssigphase und eine Gasphase erfolgt. Dabei enthält die Flüssigphase im Allgemeinen ein an oligomeren Reaktionsprodukten angereichertes Produkt, wohingegen die Gasphase an nicht umgesetzten Olefinen und gegebenenfalls gesättigten Kohlenwasserstoffen angereichert ist. Die Flüssigphase kann dann einer weiteren Auftrennung, in der Regel einer thermischen Auftrennung unter Erhalt einer die Hauptmenge des Co-Dimerisierungsprodukts enthaltenden Fraktion und gegebenenfalls weiterer Oligomerenfraktionen unterzogen werden. In einer weiteren Ausgestaltung erfolgt die direkte Auftrennung des Reaktionsaustrags (bzw. des ersten Teilstroms) durch wenigstens einen thermischen Abtrennungsschritt, vorzugsweise eine Destillation. Entspannungsschritte und/oder thermische Abtrennungsschritte können miteinander kombiniert in getrennten Vorrichtungen oder in einer einzigen Vorrichtung, z. B. einer "flash/strip-Kolonne" erfolgen.

Der bei der Aufarbeitung des Umsetzungsprodukts aus Schritt b) erhaltene, im Wesentlichen aus nicht umgesetzten Olefinen und gegebenenfalls gesättigten Kohlenwasserstoffen bestehende Strom kann gewünschtenfalls teilweise oder vollständig in die Oligomerisierungsreaktion (Schritt b)) zurückgeführt werden. Er kann zur Verhinderung der Aufpegelung inerter Komponenten auch teilweise oder vollständig aus dem System ausgeleitet werden. Mögliche Verwertungszwecke sind die Verbrennung, ein Einsatz für andere chemische Umsetzungen oder z. B. als Feed im Crack-Prozess zur erneuten Herstellung von beispielsweise in dem erfindungsgemäßen Verfahren verwertbaren Olefinen.

In einer weiteren geeigneten Ausführungsform wird ein bei der Aufarbeitung des Umsetzungsprodukts aus Schritt b) erhaltener, aus gesättigten Kohlenwasserstoffen und nicht umgesetzten Olefinen bestehender Strom einer weiteren Auftrennung, z. B. durch Rektifikation, in eine Olefin-angereicherte und eine Olefin-abgereicherte Fraktion unterzogen und die Olefin-angereicherte Fraktion zumindest teilweise in die Oligomerisierungsreaktion (Schritt b)) zurückgeführt. Die Olefin-abgereicherte Fraktion kann, wie zuvor beschrieben, aus dem Verfahren ausgeschleust und gegebenenfalls weiter verwertet werden.

Eine teilweise oder vollständige Rückführung des bei der Aufarbeitung des Umsetzungsprodukts aus Schritt b) nach Abtrennung der Co-Dimeren erhaltenen Stroms kann unabhängig von seinem Alkengehalt auch zur Steuerung des angestrebten Gehalts an Co-Dimeren sinnvoll sein.

Die Zufuhr des bei der Aufarbeitung des Umsetzungsprodukts erhaltenen, im Wesentlichen aus nicht umgesetzten Olefinen und gegebenenfalls gesättigten Kohlenwasserstoffen bestehenden Stroms kann separat oder nach vorherigem Vermischen mit einem der anderen Zuführströme erfolgen. Vor der Zufuhr in den Reaktor kann die Temperatur jedes einzelnen Stroms oder Stromgemischs mit bekannten Vorrichtungen, wie Wärmetauschern, eingestellt werden. Wird zur Oligomerisierungsreaktion ein Reaktionssystem eingesetzt, das mehrere Katalysatorzonen aufweist, so können einzelne der Ströme oder auch Gemische dieser Ströme an mehreren Stellen des Reaktionssystems eingespeist werden. Bei Verwendung einer Reaktorkaskade aus zwei oder mehr als zwei in Reihe geschalteten Reaktoren ist es möglich, einzelne Einsatzstoffe oder die vermischten Einsatzstoffströme sowohl vollständig dem ersten Reaktor der Kaskade zuzuführen oder über mehrere Zuleitungen auf die einzelnen Reaktoren der Kaskade zu verteilen.

Bei den nach dem zuvor beschriebenen Verfahren erhältlichen Olefin-Co-Dimeren handelt es sich vorzugsweise um Dimerengemische, wie sie bei der Co-Dimerisierung erhalten werden, wenn wenigstens eines der Oelfin-Einsatzmaterialien ein Gemisch aus wenigstens zwei verschiedenen Olefinen umfasst. Bevorzugt sind Olefin-Co-Dimere zu deren Herstellung wenigstens ein technisch zur Verfügung stehendes olefinhaltiges Kohlenwasserstoffgemisch als Olefin-Einsatzmaterial eingesetzt wird. Besonders bevorzugt sind Olefin-Co-Dimere, zu deren Herstellung als erstes Olefin-Einsatzmaterial ein Raffinat II und als zweites Olefin-Einsatzmaterial ein durch Dimerisierung eines Raffinats II in Gegenwart eines Nickel-haltigen Oligomerisierungskatalysators erhältliches Olefin-Einsatzmaterial eingesetzt wird.

Bevorzugt sind Olefin-Co-Dimere, die einen ISO-Index in einem Bereich von 1,0 bis 3,0, besonders bevorzugt in einem Bereich von 1,5 bis 2,5, insbesondere in einem Bereich von 1,7 bis 2,3, aufweisen.

Der ISO-Index lässt sich NMR-spektroskopisch aus den Flächenintegralen des $^1$H-NMR-Spektrums bestimmen. Dies wird wie folgt exemplarisch für $C_{12}$-Olefin-Co-Dimere (Dodecene) erläutert:

Die Dodecene werden hydriert und dadurch in Alkane der Summenformel $C_{12}$-$H_{26}$ (Dodecane) überführt. Von den Dodecanen wird ein $^1$H-NMR-Spektrum aufgenommen und blockweise integriert. Zwischen 0,3 und 1,05 ppm befinden sich die Resonanzen der $CH_3$-Protonen. Zwischen 1,05 und 2,8 ppm befinden sich die Resonanzen der $CH_2$-und CH-Protonen. Man bildet das Verhältnis der Integrale des $CH_3$-Blocks zum $CH_2$-/CH-Block. n-Dodecan weist z. B. ein Verhältnis $CH_3$-Protonen zu $CH_2$-/CH-Protonen von 6 zu 20 auf (x = 0,3). Methylundecan weist ein Verhältnis $CH_3$-Protonen zu $CH_2$-/CH-Protonen von 9 zu 17 auf (x = 0,53). Dimethyldecan weist ein Verhältnis

$CH_3$-Protonen zu $CH_2$-/CH-Protonen von 12 zu 14 auf (x = 0,86). Aus dem Verhältnis x berechnet sich der ISO-Index wie folgt:

$$\text{ISO-Index} = (20x-6)/(3x+3) \quad x = \text{Verhältnis } CH_3\text{-Protonen zu } CH_2\text{-/CH-Protonen}$$

Vorzugsweise handelt es sich bei den Olefin-Co-Dimeren um Gemische, die überwiegend oder ausschließlich Alkene mit 12 Kohlenstoffatomen enthalten. Vorzugsweise enthalten die Olefin-Co-Dimere dann wenigstens 70 Gew.-%, besonders bevorzugt wenigstens 85 Gew.-%, insbesondere wenigstens 95 Gew.-%, speziell wenigstens 95 Gew.-%, bezogen auf den Gesamtolefingehalt, Alkene mit 12 Kohlenstoffatomen.

Bevorzugt sind Olefin-Co-Dimere deren $^1$H-NMR-Spektrum im Bereich einer chemischen Verschiebung von 0,3 bis 1,05 ppm, bezogen auf Tetramethylsilan, ein Flächenintegral von 38 bis 62 %, bevorzugt 44 bis 56 %, besonders bevorzugt 47 bis 53 %, bezogen auf die Gesamtintegralfläche, aufweist.

Ein weiterer Gegenstand der Erfindung ist ein Verfahren zur Herstellung von Alkoholen, bei dem man

a) ein erstes Olefin-Einsatzmaterial bereitstellt, das im Wesentlichen aus $C_m$-Olefinen besteht und ein zweites Olefin-Einsatzmaterial durch Dimerisierung eines Raffinats II in Gegenwart eines nickelhaltigen Oligomerisierungskatalysators bereitstellt, das im Wesentlichen aus $C_n$-Olefinen besteht, wobei n und m unabhängig voneinander für zwei voneinander verschiedene ganze Zahlen von 2 bis 12 stehen, wobei das zweite Olefin-Einsatzmaterial einen Verzweigungsgrad der Olefine, bestimmt als ISO-Index, in einem Bereich von 0 bis 1,8 aufweist,

b) das erste und das zweite Olefin-Einsatzmaterial in eine erste Reaktionszone einspeist und einer Oligomerisierung an einem heterogenen Olefin-Oligomerisierungskatalysator unterzieht, der einen Nickelgehalt, bezogen auf elementares Nickel, von höchstens 1 Gew.-% aufweist und der wenigstens ein Zeolith umfasst oder aus einem Zeolith besteht,

c) aus dem Austrag der ersten Reaktionszone einen an $C_{n+m}$-Olefin-Co-Dimeren angereicherten Strom abtrennt,

d) den an $C_{n+m}$-Olefin-Co-Dimeren angereicherten Strom in eine zweite Reaktionszone einspeist und einer Umsetzung mit Kohlenmonoxid und Wasserstoff in Gegenwart eines Hydroformylierungskatalysators unterzieht, und e) hydriert.

Eine bevorzugte Ausführung ist ein Verfahren zur Herstellung von Alkoholen mit 7 bis 19 Kohlenstoffatomen, bei dem man

a) ein erstes Olefin-Einsatzmaterial bereitstellt, das im Wesentlichen aus $C_n$-Olefinen besteht und ein zweites Olefin-Einsatzmaterial durch Dimerisierung eines Raffinats II in Gegenwart eines nickelhaltigen Oligomerisierungskatalysators bereitstellt, das im Wesentlichen aus $C_{2n}$-Olefinen besteht, wobei n für eine ganze Zahl von 2 bis 6 steht, wobei das zweite Olefin-Einsatzmaterial einen Verzweigungsgrad der Olefine, bestimmt als ISO-Index, in einem Bereich von 0 bis 1,8 aufweist,

b) das erste und das zweite Olefin-Einsatzmaterial in eine erste Reaktionszone einspeist und einer Oligomerisierung an einem heterogenen Olefin-Oligomerisierungskatalysator unterzieht, der einen Nickelgehalt, bezogen auf elementares Nickel, von höchstens 1 Gew.-% aufweist und der wenigstens ein Zeolith umfasst oder aus einem Zeolith besteht,

c) aus dem Austrag der ersten Reaktionszone einen an $C_{3n}$-Olefin-Co-Dimeren angereicherten Strom abtrennt,

d) den an $C_{3n}$-Olefin-Co-Dimeren angereicherten Strom in eine zweite Reaktionszone einspeist und einer Umsetzung mit Kohlenmonoxid und Wasserstoff in Gegenwart eines Hydroformylierungskatalysators unterzieht, und

e) hydriert.

Bezüglich geeigneter und bevorzugter Ausführungsformen der Verfahrensschritte a) bis c) und der in ihnen erhaltenen Zwischenprodukte wird auf die vorherigen Ausführungen in vollem Umfang Bezug genommen.

Hydroformylierung (Schritt d))

Geeignete Katalysatoren für die Hydroformylierung in Schritt d) sind bekannt und umfassen im Allgemeinen ein Salz oder eine Komplexverbindung eines Elements der VII Nebengruppe des Periodensystems. Vorzugsweise ist das Metall der VIII. Nebengruppe ausgewählt unter Cobalt, Ruthenium, Iridium, Rhodium, Nickel, Palladium und Platin. Für das erfindungsgemäße Verfahren werden bevorzugt Salze und insbesondere Komplexverbindungen des Rhodiums oder des Cobalts verwendet.

Geeignete Salze sind beispielsweise die Hydride, Halogenide, Nitrate, Sulfate, Oxide, Sulfide oder die Salze mit Alkyl- oder Arylcarbonsäuren oder Alkyl- oder Arylsulfonsäuren. Geeignete Komplexverbindungen sind beispielsweise die Carbonylverbindungen und Carbonylhydride der genannten Metalle sowie Komplexe mit Aminen, Amiden, Triarylphosphinen, Trialkylphosphinen, Tricycloalkylphosphinen, Allyl-Aryl-Phosphinen, Olefinen, oder Dienen als Liganden. Die Liganden können auch in polymerer oder polymergebundener Form eingesetzt werden. Auch können Katalysatorsysteme in situ aus den oben genannten Salzen und den genannten Liganden hergestellt werden.

Geeignete Alkylreste der Liganden sind die zuvor beschriebenen linearen oder verzweigten $C_1$-$C_{15}$Alkyl-, insbesondere $C_1$-$C_5$-Alkylreste. Cycloalkyl steht vorzugsweise für $C_3$-$C_{10}$-Cycloalkyl, insbesondere Cyclopentyl und Cyclohexyl, die gegebenenfalls auch mit $C_1$-$C_4$-Alkylgruppen substituiert sein können. Unter Aryl versteht man vorzugsweise Phenyl (Ph) oder Naphthyl, das gegebenenfalls mit 1, 2, 3 oder 4 $C_1$-$C_4$-Alkyl, $C_1$-$C_4$-Alkoxy, z. B. Methoxy, Halogen, vorzugsweise Chlorid, oder Hydroxy, das gegebenenfalls auch ethoxyliert sein kann, substituiert ist.

Geeignete Rhodiumkatalysatoren bzw. -katalysatorvorstufen sind Rhodium(II)- und Rhodium(III)salze wie Rhodium(III)chlorid, Rhodium(III)nitrat, Rhodium(III)sulfat, Kalium-Rhodiumsulfat (Rhodiumalaun), Rhodium(II)- bzw. Rhodium(III)carboxylat, vorzugsweise Rhodium(II)- und Rhodium(III)acetat, Rhodium(II)- und Rhodium(III)-2-ethylhexanoat, Rhodium(III)oxid, Salze der Rhodium(III)säure und Trisammonium-hexachlororhodat(III).

Weiterhin eignen sich Rhodiumkomplexe der allgemeinen Formel $RhX_mL^1L^2(L^3)_n$, worin X für Halogenid, vorzugsweise Chlorid oder Bromid, Alkyl- oder Arylcarboxylat, Acetylacetonat, Aryl- oder Alkylsulfonat, insbesondere Phenylsulfonat und Toluolsulfonat, Hydrid oder das Diphenyltriazin-Anion, wobei $L^1$, $L^2$, $L^3$ unabhängig voneinander für CO, Olefine, Cycloolefine, vorzugsweise Cyclooctadien (COD), Dibenzophosphol, Benzonitril, $PR_3$ oder $R_2P$-A-$PR_2$, m für 1, 2 oder 3 und n für 0, 1 oder 2 stehen. Unter R (die Reste R können gleich oder verschieden sein) sind Alkyl-, Cycloalkyl- und Arylreste zu verstehen, vorzugsweise Phenyl, p-Tolyl, m-Tolyl, p-Ethylphenyl, p-Cumyl, p-t.-Butylphenyl, p-$C_1$-$C_4$-Alkoxyphenyl, vorzugsweise p-Anisyl, Xylyl, Mesityl, p-Hydroxyphenyl, das gegebenenfalls auch ethoxyliert vorliegen kann, Isopropyl, $C_1$-$C_4$-Alkoxy, Cyclopentyl oder Cyclohexyl. A steht für 1,2-Ethylen oder 1,3-Propylen. Bevorzugt stehen $L^1$, $L^2$ oder $L^3$ unabhängig voneinander für CO, COD, $P(Phenyl)_3$, $P(i\text{-}Propyl)_3$, $P(Anisyl)_3$, $P(OC_2H_5)_3$, $P(Cyclohexyl)_3$, Dibenzophosphol oder Benzonitril. X steht bevorzugt für Hydrid, Chlorid, Bromid, Acetat, Tosylat, Acetylacetonat oder das Diphenyltriazin-Anion, insbesondere für Hydrid, Chlorid oder Acetat.

Geeignete Cobaltverbindungen sind beispielsweise Cobalt(II)chlorid, Cobalt(II)sulfat, Cobalt(II)nitrat, deren Amin- oder Hydratkomplexe, Cobaltcarboxylate, wie Cobaltacetat, Cobaltethylhexanoat, Cobaltnaphthenoat, sowie die Carbonylkomplexe des Cobalts wie Dicobaltoctacarbonyl, Tetracobaltdodecacarbonyl und Hexacobalthexadecacarbonyl. Bevorzugt werden für das erfindungsgemäße Verfahren die Cobaltcarbonylkomplexe und insbesondere Dicobaltoctacarbonyl verwendet.

Die genannten Verbindungen des Rhodiums und Cobalts sind im Prinzip bekannt und in der Literatur hinreichend beschrieben oder sie können vom Fachmann analog zu den bereits bekannten Verbindungen hergestellt werden. Diese Herstellung kann auch in situ erfolgen, wobei die katalytisch aktive Spezies auch aus den zuvor genannten Verbindungen als Katalysatorvorstufen erst unter den Hydroformylierungsbedingungen gebildet werden kann.

Wird ein Hydroformylierungskatalysator auf Basis von Rhodium eingesetzt, so im Allgemeinen in einer Menge von 1 bis 150 ppm, bevorzugt bei 1 bis 100 ppm. Die Reaktionstemperatur liegt für einen Hydroformylierungskatalysator auf Basis von Rhodium im Allgemeinen im Bereich von Raumtemperatur bis 200 °C, bevorzugt 50 bis 170 °C.

Wird ein Hydroformylierungskatalysator auf Basis von Cobalt eingesetzt, so im Allgemeinen in einer Menge von 0,0001 bis 1,0 Gew.-%, bezogen auf die Menge der zu hydroformylierenden Olefine. Die Reaktionstemperatur liegt für einen Hydroformylierungskatalysator auf Basis von Cobalt im Allgemeinen im Bereich von etwa 80 bis 250 °C, bevorzugt von 100 bis 220 °C, besonders bevorzugt von 150 bis 200 °C.

Die Reaktion kann bei einem erhöhten Druck von etwa 10 bis 650 bar durchgeführt werden.

Das Molmengenverhältnis von $H_2$ : CO beträgt im Allgemeinen etwa 1 : 5 bis etwa 5 : 1 und vorzugsweise etwa 1 : 1.

Vorzugsweise wird in Schritt d) ein Hydroformylierungskatalysator eingesetzt, der zur Hydroformylierung linearer Monoolefine unter Erhalt eines hohen Anteils an n-Aldehyden befähigt ist.

Hydroformylierungskatalysatoren mit hoher n-Selektivität sind aus dem Stand der Technik bekannt. Dazu zählen Komplexe von Metallen der VIII. Nebengruppe mit Bis- und Polyphosphitliganden, wie sie in der US 4,668,651, der US 4,748,261, der US 4,769,498 und der US 4,885,401 beschrieben sind. Dazu zählen weiterhin Komplexe von Metallen der VIII. Nebengruppe mit zweizähnigen Phosphoratomhaltigen Liganden, die eine 1,1'-Biphenylylen-Brückengruppe

oder eine 1,1'-Binaphthylylen-Brückengruppe aufweisen, wie sie in der WO 98/19985 und der EP-A-0 937 022 beschrieben sind. Auf die genannten Liganden wird hier in vollem Umfang Bezug genommen.

Die bei der Hydroformylierung erhaltenen rohen Aldehyde bzw. Aldehyd/AlkoholGemische können vor der Hydrierung gewünschtenfalls nach üblichen, dem Fachmann bekannten Verfahren isoliert und gegebenenfalls gereinigt werden.

Hydrierung (Schritt e))

Zur Hydrierung werden die bei der Hydroformylierung erhaltenen Reaktionsgemische mit Wasserstoff in Gegenwart eines Hydrierkatalysators umgesetzt.

Geeignete Hydrierkatalysatoren sind im Allgemeinen Übergangsmetalle, wie z. B. Cr, Mo, W, Fe, Rh, Co, Ni, Pd, Pt, Ru usw. oder deren Mischungen, die zur Erhöhung der Aktivität und Stabilität auf Trägern, wie z. B. Aktivkohle, Aluminiumoxid, Kieselgur usw. aufgebracht werden können. Zur Erhöhung der katalytischen Aktivität können Fe, Co und bevorzugt Ni, auch in Form der Raney-Katalysatoren als Metallschwamm mit einer sehr großen Oberfläche verwendet werden. Bevorzugt wird für die Herstellung der erfindungsgemäßen Tensidalkohole ein Co/Mo-Katalysator eingesetzt. Die Hydrierung der Oxo-Aldehyde erfolgt in Abhängigkeit von der Aktivität des Katalysators vorzugsweise bei erhöhten Temperaturen und erhöhtem Druck. Vorzugsweise liegt die Hydriertemperatur bei etwa 80 bis 250 °C, bevorzugt liegt der Druck bei etwa 50 bis 350 bar.

Aus dem nach der Hydrierung erhaltenen Reaktionsgemisch kann nach üblichen, dem Fachmann bekannten Reinigungsverfahren, insbesondere durch fraktionierte Destillation, das Alkoholgemisch, speziell ein $C_{13}$-Alkoholgemisch, rein gewonnen werden.

Ein funktionalisiertes Alkoholgemisch kann dadurch erhalten werden, indem ein oben beschriebenes Alkoholgemisch einer

(i) Alkoxylierung,
(ii) Glycosidierung,
(iii) Sulfatierung,
(iv) Phosphatierung,
(v) Alkoxylierung und nachfolgender Sulfatierung, oder
(vi) Alkoxylierung und nachfolgender Phosphatierung

unterworfen wird.

Speziell handelt es sich um ein funktionalisiertes $C_{13}$-Alkoholgemisch.

Die Alkoxylierung der Alkoholgemische erfolgt durch Umsetzung mit mindestens einem Alkylenoxid. Bevorzugt sind die Alkylenoxide ausgewählt unter Verbindungen der allgemeinen Formel I

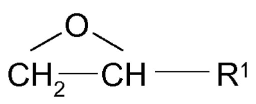

(I)

worin

$R^1$ für Wasserstoff oder einen geradkettigen oder verzweigten $C_1$- $C_{16}$-Alkylrest steht, und Mischungen davon.

Bevorzugt steht der Rest $R^1$ in der Formel I für einen geradkettigen oder verzweigten $C_1$-$C_8$-Alkylrest, insbesondere $C_1$-$C_4$-Alkylrest.

Bevorzugt sind die Alkylenoxide ausgewählt unter Ethylenoxid, Propylenoxid, Butylenoxid und Mischungen davon.

Die Umsetzung der Alkoholgemische mit dem/den Alkylenoxid(en) erfolgt nach üblichen, dem Fachmann bekannten Verfahren und in dafür üblichen Apparaturen.

Die mittlere Kettenlänge der Polyetherketten der so funktionalisierten Alkoholgemische kann durch das Molmengenverhältnis von Alkohol zu Alkylenoxid bestimmt werden. Bevorzugt werden alkoxylierte Alkoholgemische mit etwa 1 bis 200, bevorzugt etwa 1 bis 50, insbesondere 1 bis 10 Alkylenoxideinheiten hergestellt.

Die Alkoholgemische können gewünschtenfalls nur mit einem Alkylenoxid oder mit zwei oder mehreren verschiedenen Alkylenoxiden umgesetzt werden. Bei der Umsetzung der Alkoholgemische mit einem Gemisch aus zwei oder mehreren Alkylenoxiden enthalten die resultierenden Alkoxylate die Alkylenoxideinheiten im Wesentlichen statistisch verteilt. Werden die Alkylenoxide getrennt nacheinander eingesetzt, so resultieren Alkoxylate, die entsprechen der

Zugabereihenfolge die Alkylenoxideinheiten in Form von Blöcken einpolymerisiert enthalten.

Die Alkoxylierung kann durch starke Basen, wie Alkalihydroxide und Erdalkalihydroxide, Brönstedsäuren oder Lewissäuren, wie $AlCl_3$, $BF_3$ etc. katalysiert werden.

Die Alkoxylierung erfolgt vorzugsweise bei Temperaturen im Bereich von etwa 80 bis 250 °C, bevorzugt etwa 100 bis 220 °C. Der Druck liegt vorzugsweise zwischen Umgebungsdruck und 600 bar. Gewünschtenfalls kann das Alkylenoxid eine Inertgasbeimischung, z. B. von etwa 5 bis 60 %, enthalten.

Die durch Alkoxylierung erhaltenen funktionalisierten Alkoholgemische zeigen eine sehr gute Oberflächenaktivität und können als nichtionische Tenside in einer Vielzahl von Anwendungsbereichen vorteilhaft eingesetzt werden, z. B. als Tensid, Dispergiermittel, Papierhilfsmittel, Schmutzlösungsmittel, Korrosionsinhibitor, Hilfsmittel für Dispersionen oder Inkrustierungsinhibitor.

Die Glycosidierung der Alkoholgemische erfolgt durch ein-, zwei- oder mehrfache Umsetzung der erfindungsgemäßen Alkoholgemische mit Mono-, Di- oder Polysacchariden. Die Umsetzung erfolgt nach üblichen, dem Fachmann bekannten Verfahren. Dazu zählt zum einen die säurekatalysierte Umsetzung unter Wasserentzug. Geeignete Säuren sind z. B. Mineralsäuren, wie HCl und $H_2SO_4$. Dabei werden in der Regel Oligosaccharide mit statistischer Kettenlängenverteilung erhalten. Vorzugsweise liegt der durchschnittliche Oligomerisierungsgrad bei 1 bis 3 Saccharidresten. Nach einem weiteren geeigneten Verfahren kann das Saccharid zunächst durch Umsetzung mit einem niedermolekularen $C_1$-$C_8$-Alkanol, wie z. B. Ethanol, Propanol oder Butanol, acetalisiert werden. Die Acetalisierung erfolgt vorzugsweise säurekatalysiert. Das dabei resultierende Glycosid mit dem niedermolekularen Alkohol kann anschließend mit einem zuvor beschriebenen Alkoholgemisch zu den entsprechenden Glycosiden umgesetzt werden. Für diese Reaktion eignen sich im Allgemeinen auch wässrige Saccharidlösungen. Nach einem weiteren geeigneten Verfahren kann das Saccharid zunächst durch Umsetzung mit einem Halogenwasserstoff in das entsprechende O-Acetylhalosaccharid überführt und anschließend mit einem zuvor beschriebenen Alkoholgemisch in Gegenwart säurebindender Verbindungen glycosidiert werden.

Vorzugsweise werden zur Glycosidierung Monosaccharide eingesetzt. Insbesondere werden Hexosen, wie Glucose, Fructose, Galactose, Mannose etc. und Pentosen, wie Arabinose, Xylose, Ribose etc. eingesetzt. Besonders bevorzugt wird Glucose eingesetzt. Die Saccharide können einzeln oder in Form von Gemischen eingesetzt werden. Bei Saccharidgemischen resultieren im Allgemeinen Glycoside mit statistisch verteilten Zuckerresten. Bei mehrfacher Saccharidanlagerung an eine alkoholische Hydroxidgruppe resultieren Polyglycoside der zuvor beschriebenen Alkoholgemische. Auch zu Polyglycosidierung können mehrere Saccharide nacheinander oder als Gemisch eingesetzt werden, so dass die resultierenden funktionalisierten Alkoholgemische die Saccharide in Form von Blöcken oder statistisch verteilt eingebaut enthalten. Es können je nach Reaktionsbedingungen, insbesondere Reaktionstemperatur, Furanose- oder Pyranosestrukturen resultieren.

Geeignete Verfahren und Reaktionsbedingungen zur Glycosidierung sind z. B. in Ullmann's Encyclopedia of Industrial Chemistry, 5. Aufl., Bd. A25 (1994), S. 792-793 und den dort zitierten Dokumenten beschrieben.

Die durch Glycosidierung erhaltenen funktionalisierten Alkoholgemische zeigen eine sehr gute Oberflächenaktivität und können als nichtionische Tenside in einer Vielzahl von Anwendungsbereichen vorteilhaft eingesetzt werden.

Die Sulfatierung oder Phosphatierung der zuvor beschriebenen Alkoholgemische oder alkoxylierten Alkoholgemische erfolgt durch Umsetzung mit Schwefelsäure oder Schwefelsäurederivaten zu sauren Alkylsulfaten oder Alkylethersulfaten oder durch Umsetzung mit Phosphorsäure oder Phosphorsäurederivaten zu sauren Alkylphosphaten oder Alkyletherphosphaten.

Geeignete Verfahren zur Sulfatierung von Alkoholen sind die üblichen, dem Fachmann bekannten, wie sie z. B. in der US 3,462,525, US 3,420,875 oder US 3,524,864 beschrieben werden, worauf hier in vollem Umfang Bezug genommen wird. Geeignete Verfahren zur Sulfatierung sind auch in Ullmann's Encyclopedia of Industrial Chemistry, 5. Aufl., Bd. A25 (1994), S. 779-783 und der dort zitierten Literatur beschrieben.

Wird zur Sulfatierung der zuvor beschriebenen Alkoholgemische Schwefelsäure eingesetzt, so ist diese vorzugsweise 75 bis 100 gew.-%ig, insbesondere 85 bis 98 gew.-%ig. Derartige Schwefelsäure ist unter den Bezeichnungen konzentrierte Schwefelsäure und Monohydrat erhältlich.

Gewünschtenfalls kann zur Sulfatierung mit Schwefelsäure ein Lösungs- oder Verdünnungsmittel eingesetzt werden. Geeignete Lösungsmittel sind z. B. solche, die mit Wasser ein Azeotrop bilden, wie z. B. Toluol.

Nach einer geeigneten Ausführungsform zur Herstellung sulfatierter Alkoholgemische wird das Alkoholgemisch in einem Reaktionsgefäß vorgelegt und das Sulfatierungsmittel unter ständigem Durchmischen zugegeben. Zur Erzielung einer möglichst vollständigen Veresterung des Alkoholgemisches beträgt das Molmengenverhältnis von Alkanol zu Sulfatierungsmittel bevorzugt etwa 1 : 1 bis 1 : 1,5, insbesondere 1 : 1 bis 1 : 1,2. Gewünschtenfalls kann das Sulfatierungsmittel auch in einem molaren Unterschuss eingesetzt werden, z. B. bei der Sulfatierung alkoxylierter Alkoholgemische, wenn Gemische aus nichtionischen und anionischen grenzflächenaktiven Verbindungen hergestellt werden sollen. Die Sulfatierung erfolgt bevorzugt bei einer Temperatur im Bereich von Umgebungstemperatur bis 80 °C, insbesondere 40 bis 75 °C.

Weitere geeignete Sulfatierungsmittel sind z. B. Schwefeltrioxid, Schwefeltrioxid-Komplexe, Lösungen von

Schwefeltrioxid in Schwefelsäure (Oleum), Chlorsulfonsäure, Sulfurylchlorid, Amidosulfonsäure etc. Bei Einsatz von Schwefeltrioxid als Sulfatierungsmittel kann die Umsetzung vorteilhaft in einem Fallfilmverdampfer, bevorzugt im Gegenstrom, durchgeführt werden. Dabei kann die Umsetzung diskontinuierlich oder kontinuierlich erfolgen.

Die Aufarbeitung der bei der Sulfatierung entstehenden Reaktionsgemische erfolgt nach üblichen, dem Fachmann bekannten Verfahren. Dazu zählt z. B. die Neutralisierung, Abtrennung gegebenenfalls eingesetzter Lösungsmittel etc.

Die Phosphatierung der zuvor beschriebenen Alkoholgemische und alkoxylierten Alkoholgemische erfolgt im Allgemeinen in analoger Weise zur Sulfatierung.

Geeignete Verfahren zur Phosphatierung von Alkoholen sind die üblichen, dem Fachmann bekannten, wie sie z. B. in Synthesis 1985, S. 449-488 beschrieben werden, worauf hier in vollem Umfang Bezug genommen wird.

Geeignete Phosphatierungsmittel sind z. B. Phosphorsäure, Polyphosphorsäure, Phosphorpentoxid, $POCl_3$ etc. Bei Einsatz von $POCl_3$ werden die verbleibenden Säurechloridfunktionen nach der Veresterung hydrolysiert.

Die funktionalisierten Alkoholgemische eignen sich als Tenside, Dispergiermittel, Papierhilfsmittel, Schmutzlösungsmittel, Korrosionsinhibitoren, Hilfsmittel für Dispersionen, Inkrustierungsinhibitoren.

Die Erfindung wird anhand der folgenden, nicht einschränkenden Beispiele näher erläutert.

Beispiele

Beispiel 1 (erfindungsgemäß)

Ein ölbeheizter Doppelmantelrohrreaktor (d (innen) = 6 mm, l = 70 cm) wurde mit 8 ml Katalysatorsplitt (1 bis 1,6 mm Durchmesser) gefüllt. Als Katalysator wurde ein mit Aluminiumoxid (Boehmit) als Bindemittel geformter H-β-Zeolith (molares Verhältnis $SiO_2$:$Al_2O_3$ von 24) verwendet. Die Aktivierung erfolgte 18 Stunden bei 250 °C im Stickstoffstrom. Die Reaktion erfolgte bei einem Druck von 25 bar. Als Feed wurden 12 g/h einer Mischung aus 64 Gew.-% $C_8$-Olefin und 36 Gew.-% $C_4$-Olefin dosiert. Das $C_8$-Olefin mit einem ISO-Index von 1,08 entstammte einer Butendimerisierung und enthielt als Hauptkomponente 3-Methylhepten. Die Zusammensetzung des $C_4$-Olefins war wie folgt: 1-Buten (37 %), 2-Buten (42 %), iso-Buten (2 %), iso-Butan (3 %), n-Butan (16 %). Zusätzlich zum Feedstrom wurde der Reaktor mit einem Kreislaufstrom von 76 g/h durchströmt.

Die Auswertung des Versuchs erfolgte online gaschromatographisch (GC) über einen Flammionisationsdetektor, wobei die Zusammensetzung von Feed und Reaktoraustrag bestimmt wurden. In Tab. 1 ist die Differenz der GC-Flächen %-Werte (Reaktoraustrag - Feed) in Abhängigkeit der Laufzeit und der Temperatur wiedergegeben. Man erkennt deutlich, dass sowohl Butene wie Octene umgesetzt werden.

Die Austräge wurden gesammelt und destilliert. Die $C_{12}$-enthaltende Fraktion wurde hydriert und anschließend über $^1$H-NMR-Spektroskopie der ISO-Index bestimmt. Er betrug 2,1.

Das $^1$H-NMR-Spektrum der $C_{12}$-Olefine enthaltenden Fraktion weist im Bereich einer chemischen Verschiebung δ von 0,3 bis 1,05 ppm ein Flächenintegral von 51 % bezogen auf die Gesamtintegralfläche auf.

Tab. 1

| Laufzeit [h] | Temperatur [°C] | $C_4$ [Δ Fl.%] | $C_8$ [Δ Fl.%] | $C_{12}$ [Δ Fl.%] | $C_{16}$ [Δ Fl.%] |
|---|---|---|---|---|---|
| 100 | 60 | -3,87 | -4,29 | 6,25 | 1,98 |
| 160 | 80 | -5,89 | -5,73 | 8,59 | 3,01 |

Vergleichsbeispiel:

Die Durchführung erfolgte analog Beispiel 1. Als Katalysator wurden 8 ml eines geträgerten NiO/$SiO_2$-Katalysators verwendet. In Tab. 2 ist die Differenz der GC-Flächen % (Reaktoraustrag - Feed) in Abhängigkeit der Laufzeit und der Temperatur wiedergegeben. Im Gegensatz zu Beispiel 1 werden nur die Butene umgesetzt.

Tab. 2

| Laufzeit [h] | Temperatur [°C] | $C_4$ [Δ Fl.%] | Ca [Δ Fl.%] | $C_{12}$ [Δ Fl.%] | $C_{16}$ [Δ Fl.%] |
|---|---|---|---|---|---|
| 24 | 61 | -17,95 | 8,47 | 7,27 | 2,12 |
| 61 | 80 | -19,80 | 9,89 | 7,91 | 1,90 |

## Patentansprüche

1. Verfahren zur Co-Dimerisierung von Olefinen, bei dem man

   a) ein erstes Olefin-Einsatzmaterial bereitstellt, das im Wesentlichen aus $C_n$-Olefinen besteht und ein zweites Olefin-Einsatzmaterial durch Dimerisierung eines Raffinats II in Gegenwart eines nickelhaltigen Oligomerisierungskatalysators bereitstellt, das im Wesentlichen aus $C_m$-Olefinen besteht, wobei n und m unabhängig voneinander für zwei voneinander verschiedene ganze Zahlen von 2 bis 12 stehen, wobei das zweite Olefin-Einsatzmaterial einen Verzweigungsgrad der Olefine, bestimmt als ISO-Index, in einem Bereich von 0 bis 1,8 aufweist, und
   b) das erste und das zweite Olefin-Einsatzmaterial an einem heterogenen Olefin-Oligomerisierungskatalysator umsetzt, der einen Nickelgehalt, bezogen auf elementares Nickel, von höchstens 1 Gew.-% aufweist und der wenigstens ein Zeolith umfasst oder aus einem Zeolith besteht.

2. Verfahren nach Anspruch 1, bei dem man in Schritt a) ein erstes Olefin-Einsatzmaterial bereitstellt, das im Wesentlichen aus $C_n$-Olefinen besteht und ein zweites Olefin-Einsatzmaterial bereitstellt, das im Wesentlichen aus $C_{2n}$-Olefinen besteht, wobei n für eine ganze Zahl von 2 bis 6 steht.

3. Verfahren nach Anspruch 2, wobei n für 4 steht.

4. Verfahren nach einem der vorhergehenden Ansprüche, bei dem das erste Olefin-Einsatzmaterial einen Anteil an verzweigten Olefinen von höchstens 20 Gew.-%, besonders bevorzugt von höchstens 10 Gew.-%, insbesondere von höchstens 5 Gew.-%, speziell von höchstens 3 Gew.-%, bezogen auf den Gesamtolefingehalt, aufweist.

5. Verfahren nach einem der vorhergehenden Ansprüche, wobei als erstes Olefin-Einsatzmaterial ein technisch zur Verfügung stehendes olefinhaltiges Kohlenwasserstoffgemisch aus einem Crackverfahren eingesetzt wird.

6. Verfahren nach Anspruch 5, wobei als erstes Olefin-Einsatzmaterial ein Raffinat II eingesetzt wird.

7. Verfahren nach einem der vorhergehenden Ansprüche, wobei das zweite Olefin-Einsatzmaterial einen Verzweigungsgrad der Olefine, bestimmt als ISO-Index, in einem Bereich von 0,5 bis 1,5, insbesondere 0,8 bis 1,3, aufweist.

8. Verfahren nach einem der Ansprüche 1 bis 7, wobei das zweite Olefin-Einsatzmaterial nach dem DIMERSOL-Prozess oder nach dem Octol-Prozess erhalten wird.

9. Verfahren nach einem der vorhergehenden Ansprüche, wobei die Umsetzung der Olefin-Einsatzmaterialien in Schritt b) kontinuierlich erfolgt.

10. Verfahren nach einem der vorhergehenden Ansprüche, bei dem man das Umsetzungsprodukt aus Schritt b) in einen ersten und einen zweiten Teilstrom auftrennt, den ersten Teilstrom einer Aufarbeitung unter Erhalt einer im Wesentlichen das Co-Dimerisierungsprodukt enthaltenden Fraktion unterzieht und den zweiten Teilstrom in Schritt a) zurückführt.

11. Verfahren nach einem der vorhergehenden Ansprüche, wobei das molare Verhältnis von $C_m$-Olefinen zu $C_n$-Olefinen, insbesondere das molare Verhältnis von $C_{2n}$-Olefinen zu $C_n$-Olefinen, bezogen auf die in Schritt b) zugeführten Olefine, in einem Bereich von 0,25 : 1 bis 4 : 1, bevorzugt in einem Bereich von 0,5 : 1 bis 3 : 1, liegt.

12. Verfahren nach einem der vorhergehenden Ansprüche, wobei man in Schritt b) das erste und das zweite Olefin-Einsatzmaterial an einem heterogenen Olefin-Oligomerisierungskatalysator auf Basis eines Schicht- und/oder Gerüstsilicats umsetzt.

13. Verfahren nach Anspruch 12, wobei die Schicht- und/oder Gerüstsilicate des in Schritt b) eingesetzten Oligomerisierungskatalysators einen kristallinen Anteil von wenigstens 50 Gew.-% aufweisen.

14. Verfahren nach einem der Ansprüche 12 oder 13, wobei der in Schritt b) eingesetzte Oligomerisierungskatalysator wenigstens ein poröses Silicat mit einem mittleren Porendurchmesser von wenigstens 0,5 nm umfasst.

15. Verfahren zur Herstellung von Alkoholen, bei dem man

a) ein erstes Olefin-Einsatzmaterial bereitstellt, das im Wesentlichen aus Cm-Olefinen besteht und ein zweites Olefin-Einsatzmaterial durch Dimerisierung eines Raffinats II in Gegenwart eines nickelhaltigen Oligomerisierungskatalysators bereitstellt, das im Wesentlichen aus $C_n$-Olefinen besteht, wobei n und m unabhängig voneinander für zwei voneinander verschiedene ganze Zahlen von 2 bis 12 stehen, wobei das zweite Olefin-Einsatzmaterial einen Verzweigungsgrad der Olefine, bestimmt als ISO-Index, in einem Bereich von 0 bis 1,8 aufweist ;
b) das erste und das zweite Olefin-Einsatzmaterial in eine erste Reaktionszone einspeist und einer Oligomerisierung an einem heterogenen Olefin-Oligomerisierungskatalysator unterzieht, der einen Nickelgehalt, bezogen auf elementares Nickel, von höchstens 1 Gew.-% aufweist und der wenigstens ein Zeolith umfasst oder aus einem Zeolith besteht,
c) aus dem Austrag der ersten Reaktionszone einen an $C_{n+m}$-Olefin-Co-Dimeren angereicherten Strom abtrennt,
d) den an $C_{n+m}$-Olefin-Co-Dimeren angereicherten Strom in eine zweite Reaktionszone einspeist und einer Umsetzung mit Kohlenmonoxid und Wasserstoff in Gegenwart eines Hydroformylierungskatalysators unterzieht, und
e) hydriert.

**16.** Verfahren zur Herstellung von Alkoholen mit 7 bis 19 Kohlenstoffatomen, bei dem man

a) ein erstes Olefin-Einsatzmaterial bereitstellt, das im Wesentlichen aus $C_n$-Olefinen besteht und ein zweites Olefin-Einsatzmaterial durch Dimerisierung eines Raffinats II in Gegenwart eines nickelhaltigen Oligomerisierungskatalysators bereitstellt, das im Wesentlichen aus $C_{2n}$-Olefinen besteht, wobei n für eine ganze Zahl von 2 bis 6 steht, wobei das zweite Olefin-Einsatzmaterial einen Verzweigungsgrad der Olefine, bestimmt als ISO-Index, in einem Bereich von 0 bis 1,8 aufweist,
b) das erste und das zweite Olefin-Einsatzmaterial in eine erste Reaktionszone einspeist und einer Oligomerisierung an einem heterogenen Olefin-Oligomerisierungskatalysator unterzieht, der einen Nickelgehalt, bezogen auf elementares Nickel, von höchstens 1 Gew.-% aufweist und der wenigstens ein Zeolith umfasst oder aus einem Zeolith besteht,
c) aus dem Austrag der ersten Reaktionszone einen an $C_{3n}$-Olefin-Co-Dimeren angereicherten Strom abtrennt,
d) den an $C_{3n}$-Olefin-Co-Dimeren angereicherten Strom in eine zweite Reaktionszone einspeist und einer Umsetzung mit Kohlenmonoxid und Wasserstoff in Gegenwart eines Hydroformylierungskatalysators unterzieht, und
e) hydriert.

## Claims

**1.** A process for codimerizing olefins, which comprises

a) providing a first olefin starting material which consists essentially of $C_n$-olefins and providing a second olefin starting material, by dimerization of a raffinate II in the presence of a nickel-comprising oligomerization catalyst which consists essentially of $C_m$-olefins, where n and m are, independently of one another, two different integers from 2 to 12, wherein the second olefin starting material has a degree of branching of the olefins, determined as the ISO index, in the range from 0 to 1.8, and
b) reacting the first and second olefin starting materials over a heterogeneous olefin oligomerization catalyst which has a nickel content, based on elemental nickel, of not more than 1% by weight and which comprises at least one zeolite or consists of a zeolite.

**2.** The process according to claim 1, wherein, in step a), a first olefin starting material which consists essentially of $C_n$-olefins is provided and a second olefin starting material which consists essentially of $C_{2n}$-olefins, where n is an integer from 2 to 6, is provided.

**3.** The process according to claim 2, wherein n is 4.

**4.** The process according to any of the preceding claims, wherein the first olefin starting material has a proportion of branched olefins of not more than 20% by weight, particularly preferably not more than 10% by weight, in particular not more than 5% by weight, especially not more than 3% by weight, based on the total olefin content.

**5.** The process according to any of the preceding claims, wherein an industrially available olefin-comprising hydrocarbon

mixture from a cracking process is used as first olefin starting material.

**6.** The process according to claim 5, wherein a raffinate II is used as first olefin starting material.

**7.** The process according to any of the preceding claims, wherein the second olefin starting material has a degree of branching of the olefins, determined as the ISO index, in the range from 0 to 1.8, preferably from 0.5 to 1.5, in particular from 0.8 to 1.3.

**8.** The process according to any of claims 1 to 7, wherein the second olefin starting material is obtained by the DIMERSOL process or by the Octol process.

**9.** The process according to any of the preceding claims, wherein the reaction of the olefin starting materials in step b) is carried out continuously.

**10.** The process according to any of the preceding claims, wherein the reaction product from step b) is separated into a first substream and a second substream, the first substream is subjected to a work-up to give a fraction comprising essentially the codimerization product and the second substream is recirculated to step a).

**11.** The process according to any of the preceding claims, wherein the molar ratio of $C_m$-olefins to $C_n$-olefins, in particular the molar ratio of $C_{2n}$-olefins to $C_n$-olefins, based on the olefins fed in in step b), is in the range from 0.25:1 to 4:1, preferably in the range from 0.5:1 to 3:1.

**12.** The process according to any of the preceding claims, wherein the first olefin starting material and the second olefin starting material are reacted over a heterogeneous olefin oligomerization catalyst based on a sheet and/or framework silicate in step b).

**13.** The process according to claim 12, wherein the sheet and/or framework silicates of the oligomerization catalyst used in step b) have a proportion of crystalline material of at least 50% by weight.

**14.** The process according to any of claims 12 and 13, wherein the oligomerization catalyst used in step b) comprises at least one porous silicate having a mean pore diameter of at least 0.5 nm.

**15.** A process for preparing alcohols, which comprises

a) providing a first olefin starting material which consists essentially of $C_m$-olefins and providing a second olefin starting material, by dimerization of a raffinate II in the presence of a nickel-comprising oligomerization catalyst which consists essentially of $C_n$-olefins, where n and m are, independently of one another, two different integers from 2 to 12; wherein the second olefin starting material has a degree of branching of the olefins, determined as the ISO index, in the range from 0 to 1.8, and
b) feeding the first and second olefin starting materials into a first reaction zone and subjecting them to an oligomerization over a heterogeneous olefin oligomerization catalyst which has a nickel content, based on elemental nickel, of not more than 1% by weight and which comprises at least one zeolite or consists of a zeolite,
c) separating off a stream enriched in $C_n$+m-olefin codimers from the discharge from the first reaction zone,
d) feeding the stream enriched in $C_{n+m}$-olefin codimers into a second reaction zone and subjecting it to a reaction with carbon monoxide and hydrogen in the presence of a hydroformylation catalyst and
e) hydrogenating the hydroformylation product.

**16.** A process for preparing alcohols having from 7 to 19 carbon atoms, which comprises

a) providing a first olefin starting material which consists essentially of $C_n$-olefins and providing a second olefin starting material, by dimerization of a raffinate II in the presence of a nickel-comprising oligomerization catalyst which consists essentially of $C_{2n}$-olefins, where n is an integer from 2 to 6, wherein the second olefin starting material has a degree of branching of the olefins, determined as the ISO index, in the range from 0 to 1.8,
b) feeding the first and second olefin starting materials into a first reaction zone and subjecting them to an oligomerization over a heterogeneous olefin oligomerization catalyst which has a nickel content, based on elemental nickel, of not more than 1% by weight and which comprises at least one zeolite or consists of a zeolite,
c) separating off a stream enriched in $C_{3n}$-olefin codimers from the discharge from the first reaction zone,
d) feeding the stream enriched in $C_{3n}$-olefin codimers into a second reaction zone and subjecting it to a reaction

with carbon monoxide and hydrogen in the presence of a hydroformylation catalyst and
e) hydrogenating the hydroformylation product.

## Revendications

1. Procédé pour la codimérisation d'oléfines, dans lequel

a) on met à disposition un premier matériau oléfinique de départ, qui est essentiellement constitué par des oléfines en $C_n$, et un deuxième matériau oléfinique de départ par dimérisation d'un raffinat II en présence d'un catalyseur d'oligomérisation contenant du nickel, qui est essentiellement constitué par des oléfines en $C_m$, n et m représentant, indépendamment l'un de l'autre, deux nombres entiers différents l'un de l'autre de 2 à 12, le deuxième matériau oléfinique de départ présentant un degré de ramification des oléfines, déterminé en tant qu'indice ISO, dans une plage de 0 à 1, 8, et
b) on transforme le premier et le deuxième matériau oléfinique de départ sur un catalyseur d'oligomérisation d'oléfines, hétérogène, qui présente une teneur en nickel, par rapport au nickel élémentaire, d'au plus 1% en poids et qui comprend au moins une zéolithe ou qui est constitué par une zéolithe.

2. Procédé selon la revendication 1, dans lequel, dans l'étape a), on met à disposition un premier matériau oléfinique de départ, qui est essentiellement constitué par des oléfines en $C_n$, et un deuxième matériau oléfinique de départ, qui est essentiellement constitué par des oléfines en $C_{2n}$, n représentant un nombre entier 2 à 6.

3. Procédé selon la revendication 2, n valant 4.

4. Procédé selon l'une quelconque des revendications précédentes, dans lequel le premier matériau oléfinique de départ présente une proportion d'oléfines ramifiées d'au plus 20% en poids, de manière particulièrement préférée d'au plus 10% en poids, en particulier d'au plus 5% en poids, tout particulièrement d'au plus 3% en poids, par rapport à la teneur totale en oléfines.

5. Procédé selon l'une quelconque des revendications précédentes, un mélange d'hydrocarbures contenant des oléfines provenant d'un procédé de craquage, industriellement à disposition, étant utilisé comme premier matériau oléfinique de départ.

6. Procédé selon la revendication 5, un raffinat II étant utilisé comme premier matériau oléfinique de départ.

7. Procédé selon l'une quelconque des revendications précédentes, le deuxième matériau oléfinique de départ présentant un degré de ramification des oléfines, déterminé en tant qu'indice ISO, dans une plage de 0,5 à 1,5, en particulier de 0,8 à 1, 3.

8. Procédé selon l'une quelconque des revendications 1 à 7, le deuxième matériau oléfinique de départ étant obtenu selon le procédé DIMERSOL ou le procédé Octol.

9. Procédé selon l'une quelconque des revendications précédentes, la transformation des matériaux oléfiniques de départ dans l'étape b) ayant lieu de manière continue.

10. Procédé selon l'une quelconque des revendications précédentes, dans lequel on sépare le produit de transformation provenant de l'étape b) en un premier et un deuxième flux partiel, on soumet le premier flux partiel à un traitement avec obtention d'une fraction contenant essentiellement le produit de codimérisation et on recycle le deuxième flux partiel dans l'étape a).

11. Procédé selon l'une quelconque des revendications précédentes, le rapport molaire des oléfines en $C_m$ aux oléfines en $C_n$, en particulier le rapport molaire des oléfines en $C_{2n}$ aux oléfines en $C_n$, par rapport aux oléfines introduites dans l'étape b), étant situé dans une plage de 0,25:1 à 4:1, de préférence dans une plage de 0,5:1 à 3:1.

12. Procédé selon l'une quelconque des revendications précédentes, le premier et le deuxième matériau oléfinique de départ étant transformés, dans l'étape b), sur un catalyseur d'oligomérisation d'oléfines, hétérogène, à base d'un silicate à couches et/ou d'un silicate structuré.

**13.** Procédé selon la revendication 12, les silicates à couches et/ou les silicates structurés du catalyseur d'oligomérisation utilisé dans l'étape b) présentant une proportion cristalline d'au moins 50% en poids.

**14.** Procédé selon l'une quelconque des revendications 12 ou 13, le catalyseur d'oligomérisation utilisé dans l'étape b) comprenant au moins un silicate poreux présentant un diamètre moyen des pores d'au moins 0,5 nm.

**15.** Procédé pour la préparation d'alcools, dans lequel

a) on met à disposition un premier matériau oléfinique de départ, qui est essentiellement constitué par des oléfines en $C_m$, et un deuxième matériau oléfinique de départ par dimérisation d'un raffinat II en présence d'un catalyseur d'oligomérisation contenant du nickel, qui est essentiellement constitué par des oléfines en $C_n$, n et m représentant, indépendamment l'un de l'autre, deux nombres entiers différents l'un de l'autre de 2 à 12, le deuxième matériau oléfinique de départ présentant un degré de ramification des oléfines, déterminé en tant qu'indice ISO, dans une plage de 0 à 1, 8,
b) on injecte le premier et le deuxième matériau oléfinique de départ dans une première zone de réaction et on les soumet à une oligomérisation sur un catalyseur d'oligomérisation d'oléfines, hétérogène, qui présente une teneur en nickel, par rapport au nickel élémentaire, d'au plus 1% en poids et qui comprend au moins une zéolithe ou qui est constitué par une zéolithe,
c) on sépare du produit sorti de la première zone de réaction un flux enrichi en codimères d'oléfines en $C_{n+m}$,
d) on injecte le flux enrichi en codimères d'oléfines en $C_{n+m}$ dans une deuxième zone de réaction et on les soumet à une transformation avec du monoxyde de carbone et de l'hydrogène en présence d'un catalyseur d'hydroformylation et
e) on hydrogène.

**16.** Procédé pour la préparation d'alcools comprenant 7 à 19 atomes de carbone, dans lequel

a) on met à disposition un premier matériau oléfinique de départ, qui est essentiellement constitué par des oléfines en $C_n$, et un deuxième matériau oléfinique de départ par dimérisation d'un raffinat II en présence d'un catalyseur d'oligomérisation contenant du nickel, qui est essentiellement constitué par des oléfines en $C_{2n}$, n représentant un nombre entier de 2 à 6, le deuxième matériau oléfinique de départ présentant un degré de ramification des oléfines, déterminé en tant qu'indice ISO, dans une plage de 0 à 1,8,
b) on injecte le premier et le deuxième matériau oléfinique de départ dans une première zone de réaction et on les soumet à une oligomérisation sur un catalyseur d'oligomérisation d'oléfines, hétérogène, qui présente une teneur en nickel, par rapport au nickel élémentaire, d'au plus 1% en poids et qui comprend au moins une zéolithe ou qui est constitué par une zéolithe,
c) on sépare du produit sorti de la première zone de réaction un flux enrichi en codimères d'oléfines en $C_{3n}$,
d) on injecte le flux enrichi en codimères d'oléfines en $C_{3n}$ dans une deuxième zone de réaction et on les soumet à une transformation avec du monoxyde de carbone et de l'hydrogène en présence d'un catalyseur d'hydroformylation et
e) on hydrogène.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0053547 A
- DE 19859911 A
- WO 0056683 A
- WO 0136356 A
- US 3402217 A
- EP 0329305 A
- US 4029719 A
- CA 1205792
- US 20040220440 A1
- WO 2004080935 A
- WO 03082780 A
- WO 0183407 A
- US 5118901 A
- WO 2007040812 A
- EP 81041 A
- DE 1568542
- DE 19845857 A
- US 4668651 A
- US 4748261 A
- US 4769498 A
- US 4885401 A
- WO 9819985 A
- EP 0937022 A
- US 3462525 A
- US 3420875 A
- US 3524864 A


### In der Beschreibung aufgeführte Nicht-Patentliteratur


- *Revue de l'Institut Français du Petrole,* September 1982, vol. 37 (5), 639
- **Z. M. THOMAS ; W. Z. THOMAS.** *Principles and Practice of Heterogeneous-Catalysis,* 1997, ISBN 3-527-29239-8, 347
- Ullmann's Encyclopedia of Industrial Chemistry. 1994, vol. A25, 792-793
- Ullmann's Encyclopedia of Industrial Chemistry. 1994, vol. A25, 779-783
- *Synthesis,* 1985, 449-488